# EUROPEAN PATENT SPECIFICATION

(11) **EP 4 308 108 B1**
(45) Date of publication and mention of the grant of the patent: **09.12.2026**
(21) Application number: 22771972.1
(22) Date of filing: 11.03.2022
(51) Int. Cl.: A61K 31/395, A61K 31/40, A61K 31/407, C07D 471/04

(54) **POLYHYDROXYLATED INDOLIZIDINE AND PYRROLIZIDINE DERIVATIVES AND USES THEREOF**
POLYHYDROXYLIERTE INDOLIZIDIN- UND PYRROLIZIDINDERIVATE UND IHRE VERWENDUNG
DÉRIVÉS D'INDOLIZIDINE ET DE PYRROLIZIDINE POLYHYDROXYLÉS ET LEURS UTILISATIONS

(30) Priority: 16.03.2021 US 202163161580 P
(43) Date of publication of application: 24.01.2024
(73) Proprietor: Academia Sinica, Taipei 115 (TW)
(72) Inventor: CHENG, Wei-Chieh, Taipei City 105 (TW); CHEN, Wei-An, Taitung City Taitung County 950 (TW); CHEN, Yu-Hsin, Chiayi County 621 (TW); CHENG, Ting-Jen, New Taipei City 234 (TW); SHEN, Chia-Ning, Keelung City 206 (TW); HSIEH, Chiao-Yun, New Taipei City 231071 (TW); HUNG, Pi-Fang, Taoyuan City 333006 (TW)
(74) Representative: Lang, Christian
(86) International application number: PCT/US2022/020074
(87) International publication number: WO 2022/197564

(56) References cited:
- WO-A2-2010/049678
- US-A- 5 466 809
- US-A1- 2002 099 065
- PLUVINAGE BENJAMIN ET AL: "Inhibition of the exo-[beta]-d-glucosaminidase CsxA by a glucosamine-configured castanospermine and an amino-australine analogue", ORGANIC & BIOMOLECULAR CHEMISTRY, vol. 7, no. 20, 14 August 2009 (2009-08-14), pages 4169 - 4172, XP093233730, ISSN: 1477-0520, DOI: 10.1039/b913235j
- RIBES CELIA ET AL: "Stereoselective Synthesis of the Glycosidase Inhibitor Australine through a One-Pot, Double-Cyclization Strategy", ORGANIC LETTERS, vol. 9, no. 1, 6 December 2006 (2006-12-06), US, pages 77 - 80, XP093233737, ISSN: 1523-7060, DOI: 10.1021/ol062570v
- GERBER-LEMAIRE SANDRINE ET AL: "Glycosylation Pathways as Drug Targets for Cancer: Glycosidase Inhibitors", MINI-REVIEWS IN MEDICINAL CHEMISTRY, vol. 6, no. 9, 1 September 2006 (2006-09-01), NL, pages 1043 - 1052, XP093233750, ISSN: 1389-5575, DOI: 10.2174/138955706778195162
- TANG M ET AL: "Asymmetric synthesis of (+)-1-epiaustraline and attempted synthesis of australine", TETRAHEDRON, ELSEVIER SIENCE PUBLISHERS, AMSTERDAM, NL, vol. 60, no. 27, 28 June 2004 (2004-06-28), pages 5759 - 5767, XP004516476, ISSN: 0040-4020, DOI: 10.1016/J.TET.2004.05.010
- TANG M ET AL: "Asymmetric synthesis of (-)-7-epiaustraline and (+)-1,7-diepiaustraline", THE JOURNAL OF ORGANIC CHEMISTRY, AMERICAN CHEMICAL SOCIETY, UNITED STATES, vol. 68, no. 20, 1 January 2003 (2003-01-01), pages 7818 - 7824, XP002348524, ISSN: 0022-3263, DOI: 10.1021/JO034914Q
- PEARSON W H ET AL: "Preparation of immobilized swainsonine analogs on solid support", TETRAHEDRON LETTERS, ELSEVIER, AMSTERDAM, NL, vol. 43, no. 12, 18 March 2002 (2002-03-18), pages 2175 - 2178, XP027241819, ISSN: 0040-4039, [retrieved on 20020318]
- DATABASE REGISTRY [online] CHEMICAL ABSTRACTS SERVICE, COLUMBUS, OHIO, US; 4 December 2015 (2015-12-04), ANONYMOUS, XP093233851, Database accession no. 1822453-35-9
- FURNEAUX RICHARD H ET AL: "The Chemistry of Castanospermine, Part I: Synthetic Modifications at C-6", TETRAHEDRON, vol. 50, no. 7, 1 January 1994 (1994-01-01), pages 2131 - 2160, XP093233929, DOI: org/10.1016/S0040-4020(01)85075-4
- DATABASE Pubchem substance ANONYMOUS : "SID 127423347 ", XP055971992
- CHEN WEI-AN, CHEN YU-HSIN, HSIEH CHIAO-YUN, HUNG PI-FANG, CHEN CHIAO-WEN, CHEN CHIEN-HUNG, LIN JUNG-LEE, CHENG TING-JEN R., HSU TS: "Harnessing natural-product-inspired combinatorial chemistry and computation-guided synthesis to develop N -glycan modulators as anticancer agents", CHEMICAL SCIENCE, ROYAL SOCIETY OF CHEMISTRY, UNITED KINGDOM, vol. 13, no. 21, 1 June 2022 (2022-06-01), United Kingdom , pages 6233 - 6243, XP055971986, ISSN: 2041-6520, DOI: 10.1039/D1SC05894K

## Description

### BACKGROUND OF THE INVENTION

### 1. FIELD OF THE INVENTION

The present disclosure in general relates to polyhydroxylated indolizidine and pyrrolizidine derivates, in particular to polyhydroxylated indolizidine and pyrrolizidine derivates that selectively inhibit human Golgi α-mannosidase II (α-hGMII) over human α-mannosidase (α-hLM) enzyme activity.

### 2. DESCRIPTION OF RELATED ART

Protein glycosylation is the post-translational process and is also important for a lot of cellular functions. Indeed, glycans play an important role in intercellular and intracellular processes, including cell adhesion and development, cell recognition, and cancer development and metastasis, and also controls and defines fundamental biological processes directing crucial physiological functions. Further, modulations of glycans enable to affect cell-cell communications and recognitions, and even pathogen-cell interactions. Most of them are associated with human diseases including cancer, autoimmune disease, viral infection, and even Alzheimer's disease. Therefore, developing potent and selective small molecule-based inhibitors to target a specific sugar processing enzyme including glycol-transferase or glycosidase to modulate a glycosylation pathway is an attractive and promising therapeutic approach.

Among the enzymes involved in the mammalian N-glycosylation pathway, Golgi *α-*mannosidase II (α-hGMII) plays an important role to affect cellular glycoforms. Inhibition of its function on cancer cells will change patterns of the N-linked oligosaccharides, highly correlated to downregulate the tumor progression, metastasis, or invasion and also to modulate the immune response
Several naturally occurring or synthetic mannosidase inhibitors have been developed by us and others. For instance, an alkaloid - swainsonine, with a potent inhibitory activity against mannosidase has been reported. For example, document US 2002/099065 A1 discloses analogues of swainsonine, processes for their preparation and their use as therapeutic agents. However, one difficulty in utilizing swainsonine is that it suffers from a lack of selectivity between lysosomal α-mannosidase (α-hLM) (EC 3.2.1.24) and α-hGMII. Inhibition of α-hLM by swainsonine causes the accumulation of undegraded mannose-containing carbohydrate in lysosome that limits its clinical study *(*Clin. Cancer Res. 1995, 1, 935 and J. Cell Biol. 1985, 101, 339*).*

Accordingly, there exist in the related art a need of a novel α-hGMII inhibitor, which has a selectivity toward α-hGMII than to α-hLM, and is also effective in suppressing the growth of cancer cells *in vitro* and *in vivo.*

### SUMMARY OF THE INVENTION

This invention is set out in the appended set of claims. The present disclosure provides novel compounds capable of selectively suppressing Golgi α-mannosidase II (α-hGMII) over α-mannosidase (α-hLM) enzyme activity and growth of cancer cells both *in vitro* and *in vivo.* A first aspect of the present disclosure is directed to a compound of formula (I), 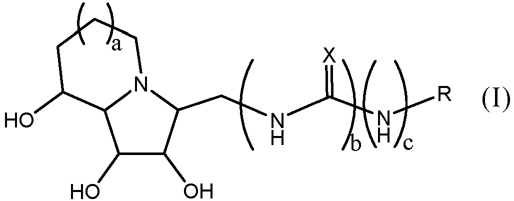
a salt, or a solvate thereof,
wherein:
   X is O or S;
   a, b and c are independently an integral of 0 or 1; and
   R is selected from the group consisting of H, C₁₋₆ alkyl, alkenyl, alkynyl, aryl, heteroaryl, cycloalkyl, cycloalkenyl, aralkyl, aralkenyl, aralkynyl, heteroaralkyl, heteroaralkenyl, heteroaralkynyl, heterocyclyl, alkoxy, aryloxy, and sulfonyl.

In certain examples not forming part of the claims, the compound has the structure of formula (II), 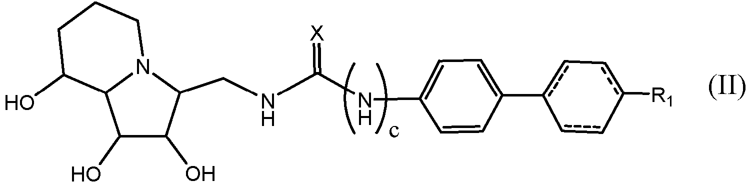 wherein,
- - - - - - is a single or double bond;
X is O or S;
c is 0 or 1; and
R₁ is hydrogen or C₁₋₆ alkyl.

According to the present invention, the compound is selected from the group consisting of:

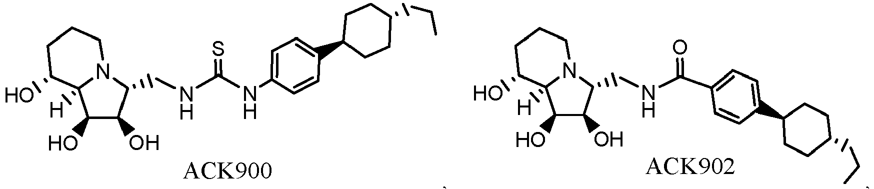

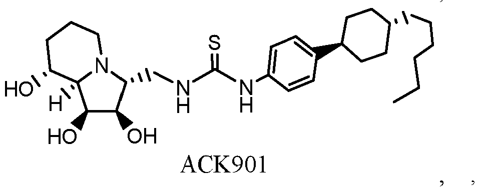

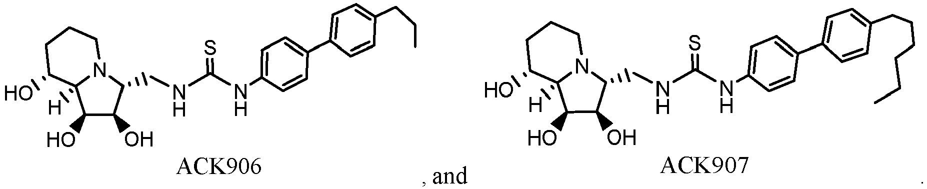

In certain examples not forming part of the claims, the compound has the structure of formula (III), 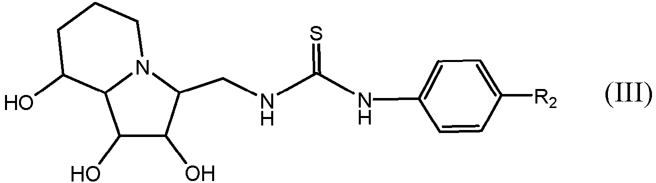 wherein,
R₂ is hydrogen, C₁₋₆ alkyl, or alkoxy.

In some examples not forming part of the claims, the compound of formula (III) is any one of,

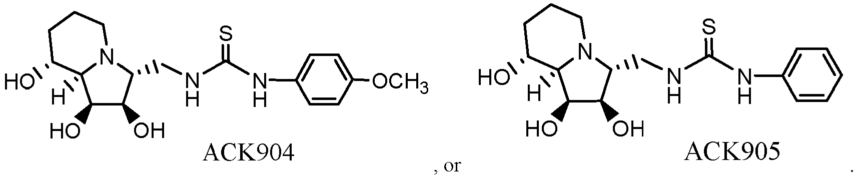

In further embodiments, the compound is,

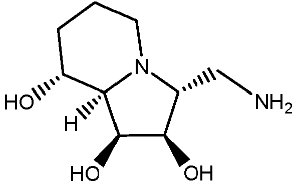

It is the second aspect of the present disclosure to provide a pharmaceutical composition, which comprises a compound of the present invention, or a pharmaceutically acceptable salt thereof, and a pharmaceutically acceptable excipient.

It is the third aspect of the present disclosure to provide said pharmaceutical composition for use in treating a subject having a cancer by administering to the subject an effective amount of any one of the compounds according to the present invention.

Examples of cancer that may be treated by use of the pharmaceutical composition of the present invention include, but are not limited to, cancer is selected from the group consisting of bone tumor, brain cancer, breast cancer, cervical cancer, CNS neoplasm, colon cancer, esophageal cancer, Ewing's sarcoma, head and neck cancer, Hodgkin's disease, larynx cancer, leukemia, liver cancer, lymphoma, melanoma, multiple myeloma, nasopharynx cancer, neuroblastoma, non-small-cell lung (NSCL) cancer, pancreatic cancer, prostate cancer, rectal cancer, retinoblastoma, small-cell lung (SCL) cancer, testicular cancer, thyroid cancer, skin cancer other than melanoma, and Wilms' tumor.

In some embodiments of the present disclosure, the cancer is a metastatic cancer.

In all embodiments, the subject is a human.

The details of one or more embodiments of this disclosure are set forth in the accompanying description below. Other features and advantages of the invention will be apparent from the detail descriptions, and from claims.

It is to be understood that both the foregoing general description and the following detailed description are by examples, and are intended to provide further explanation of the invention as claimed.

### BRIEF DESCRIPTION OF THE DRAWINGS

The patent or application file contains at least one drawing executed in colors. Copies of this patent or patent application publication with color drawing(s) will be provided by the Office upon request and payment of the necessary fee.

The accompanying drawings, which are incorporated in and constitute a part of the specification, illustrate various example systems, methods and other exemplified embodiments of various aspects of the invention. The present description will be better understood from the following detailed description read in light of the accompanying drawings, where,

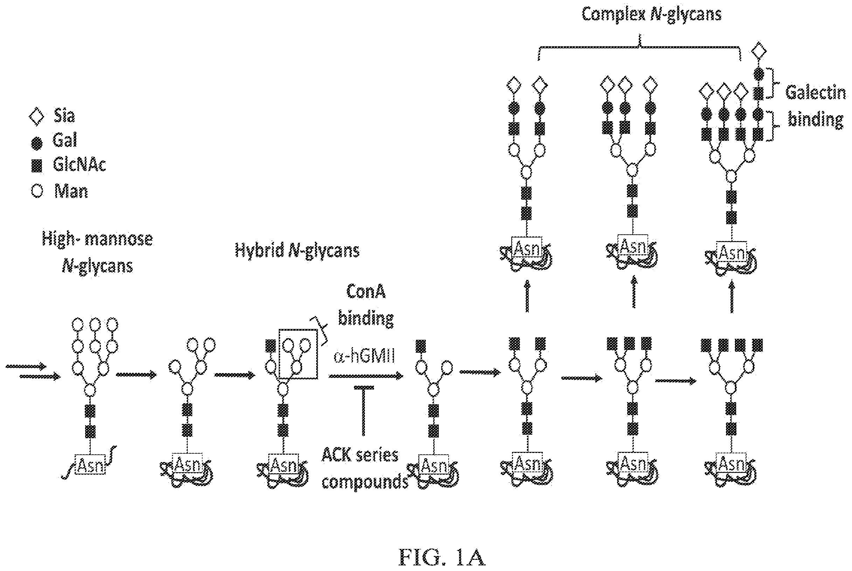

FIG 1A is a schematic representation of the mammalian N-glycan synthesis pathway;

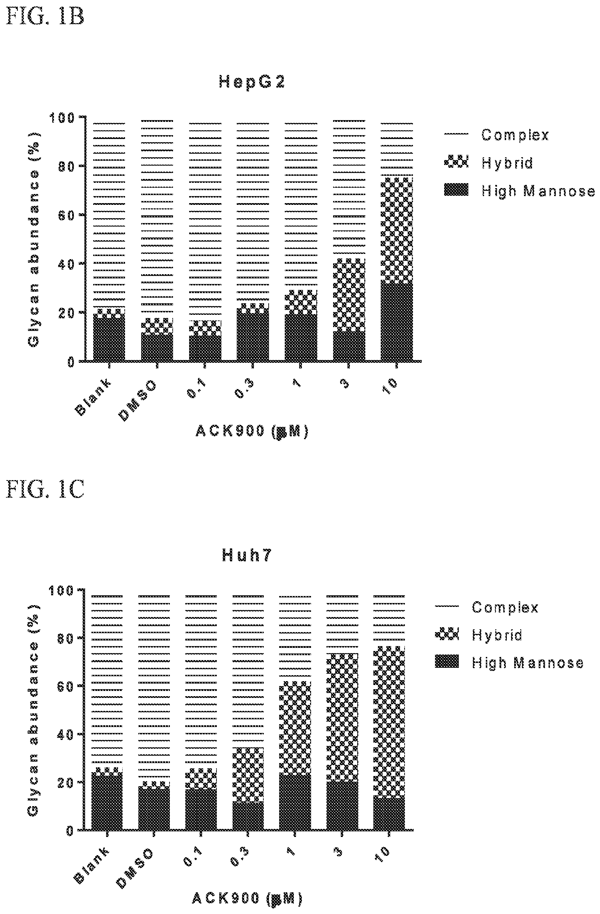

FIGs 1B and 1C are bar graphs respectively depicting the effect of ACK900 on *N-*glycan abundance of (B) HepG2 and (C) Huh7 cells in accordance with one embodiment of the present disclosure;

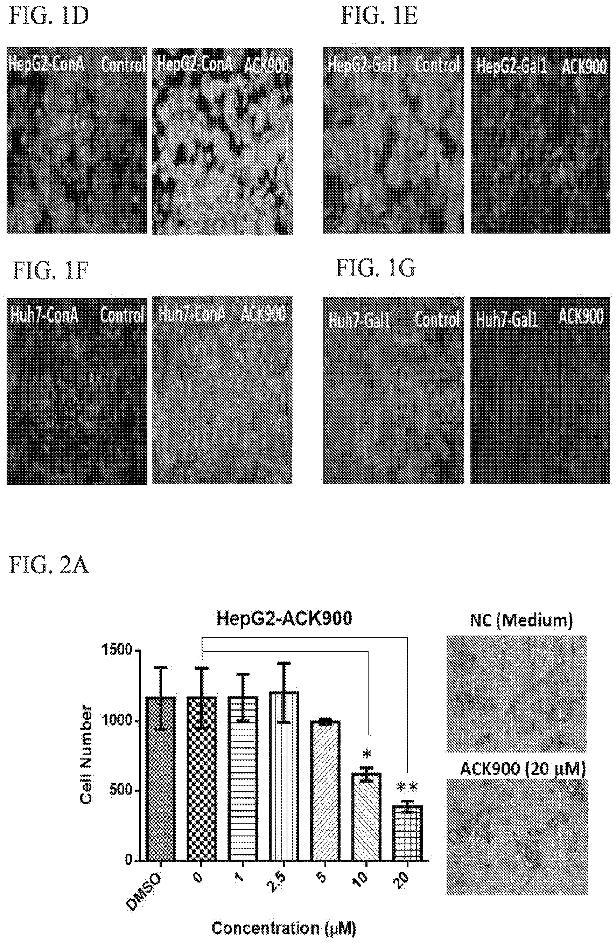

FIGs 1D to 1G are photos of microscope images of untreated and ACK900-treated HepG2 cells stained with (D) ConA-FITC (green) and (E) Alexa Fluor 555 conjugated Gal1 (Gal1-555, red), and Huh7 cells stained with (F) ConA-FITC and (G) Gal1-555 in accordance with one embodiment of the present disclosure;

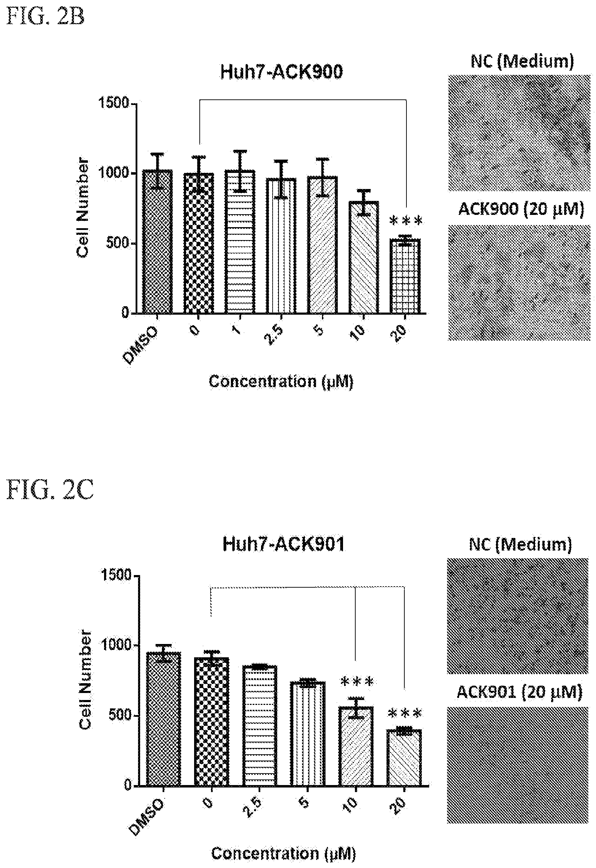

FIGs 2A and 2B respectively depict the effects of ACK900 on cell migration of (A) HepG2, and **(B)** Huh7 cells in accordance with one embodiment of the present disclosure. Results are presented as means of three independents experiments. *P<0.05, **P<0.01, ***P<0.001, P<0.05 is considered statistically significant;

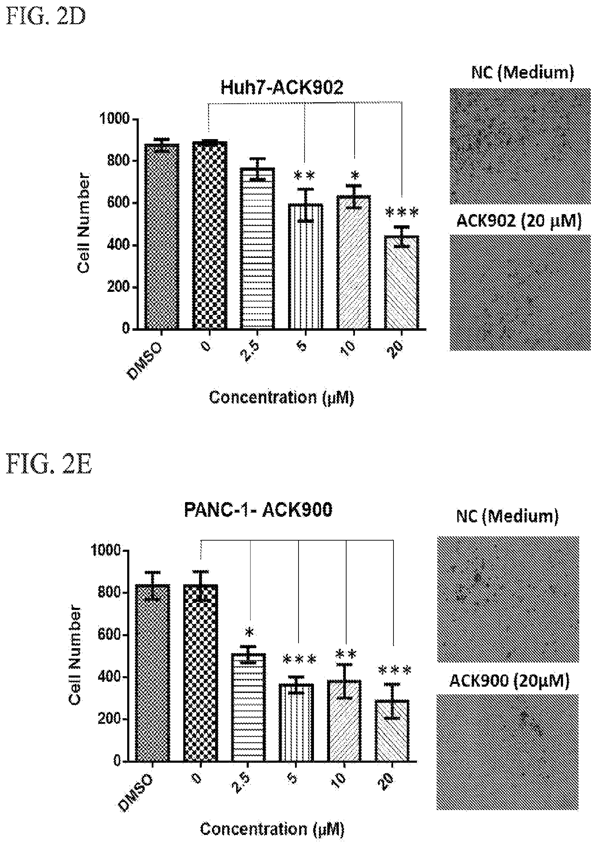

**FIGs 2C** **and** **2D** respectively depict the effects of **(C) ACK901** and **(D) ACK902** on cell migration of Huh7 cells in accordance with one embodiment of the present disclosure. Results are presented as means of three independents experiments. *P<0.05, **P<0.01, ***P<0.001, P<0.05 is considered statistically significant;

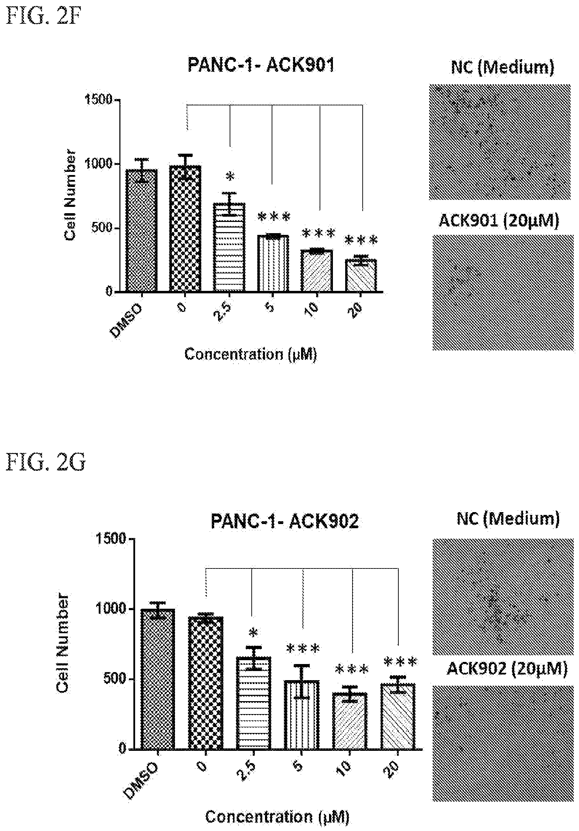

**FIGs 2E** **and** **2F** respectively depict the effects of **(E) ACK900** and **(F) ACK901** on cell migration of PANC-1 cells in accordance with one embodiment of the present disclosure. Results are presented as means of three independents experiments. *P<0.05, **P<0.01, ***P<0.001, P<0.05 is considered statistically significant;
**FIG 2G** depicts the effect of **ACK902** on cell migration of PANC-1 cells in accordance with one embodiment of the present disclosure. Results are presented as means of three independents experiments. *P<0.05, **P<0.01, ***P<0.001, P<0.05 is considered statistically significant;

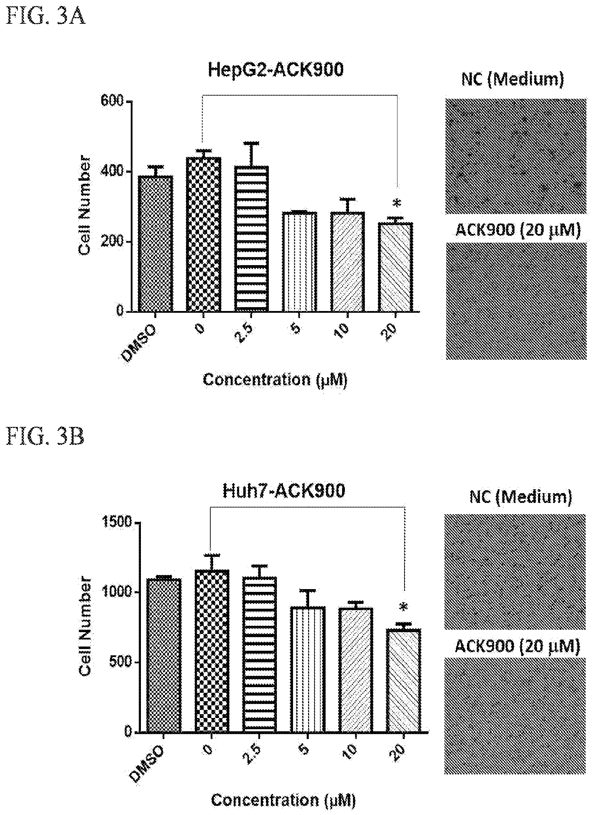

**FIGs 3A** and 3B respectively depict the effects of **ACK900** on cell invasion of **(A)** HepG2, and **(B)** Huh7 cells in accordance with one embodiment of the present disclosure. Results are presented as means of three independents experiments. *P<0.05, **P<0.01, ***P<0.001, P<0.05 is considered statistically significant;

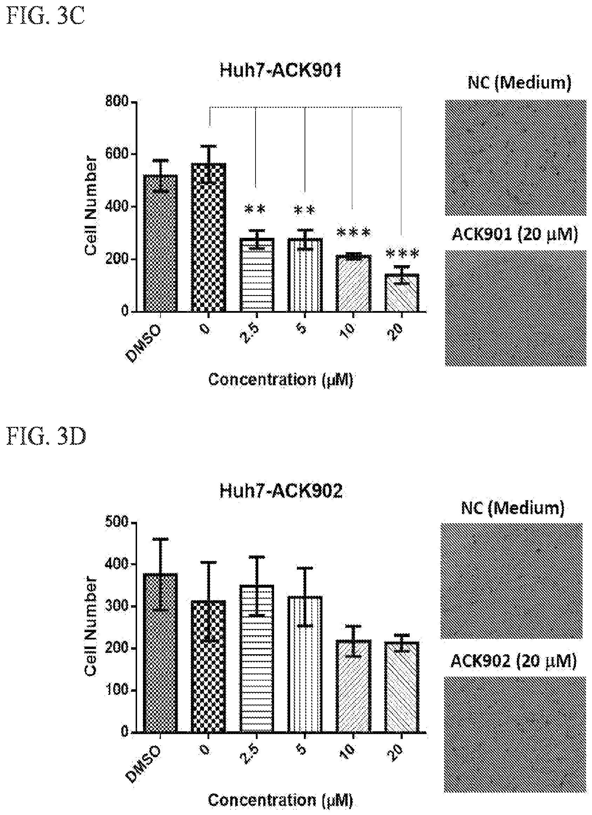

**FIGs 3C and 3D** respectively depict the effects of **(C) ACK901** and **(D) ACK902** on cell invasion of Huh7 cells in accordance with one embodiment of the present disclosure. Results are presented as means of three independents experiments. *P<0.05, **P<0.01, ***P<0.001, P<0.05 is considered statistically significant;

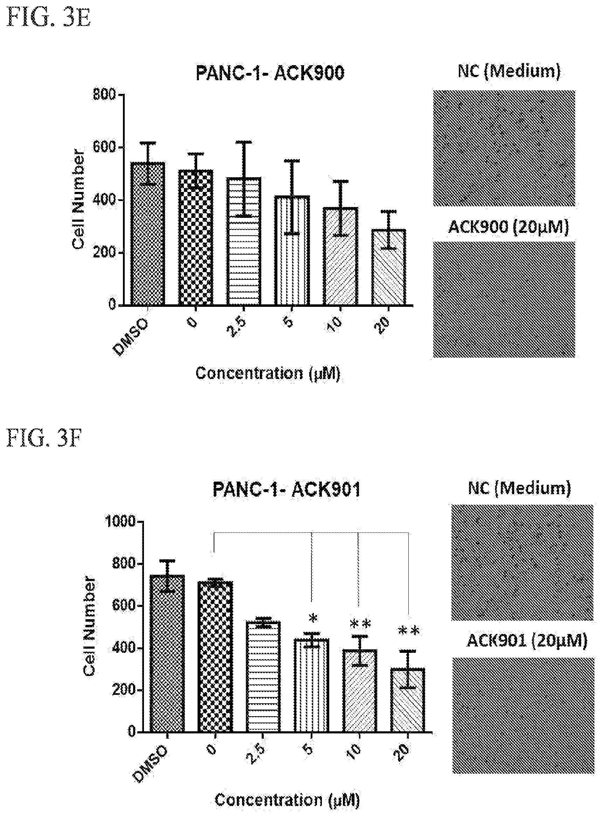

**FIGs 3E and 3F** respectively depict the effects of **(E) ACK900** and **(F) ACK901** on cell invasion of PANC-1 cells in accordance with one embodiment of the present disclosure. Results are presented as means of three independents experiments. *P<0.05, **P<0.01, ***P<0.001, P<0.05 is considered statistically significant;

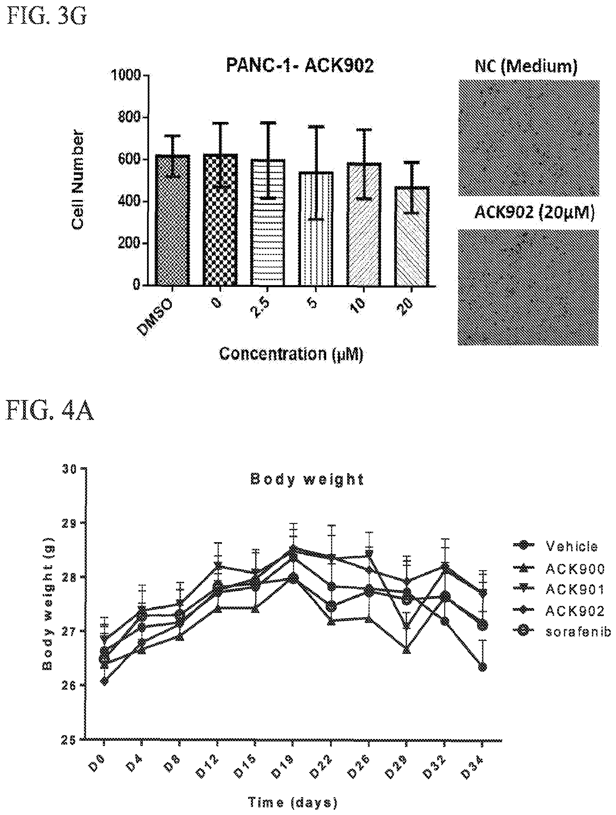

**FIG 3G** depicts the effect of **ACK902** on cell invasion of PANC-1 cells in accordance with one embodiment of the present disclosure. Results are presented as means of three independents experiments. *P<0.05, **P<0.01, ***P<0.001, P<0.05 is considered statistically significant;
**FIGs 4A to 4C** respectively depict the in vivo effects of **ACK900, ACK901, and ACK902** on **(A)** the body weight, **(B)** the size (mm³) of tumor, and **(C)** the tumor weight of mice grafted with HCC in accordance with one embodiment of the present disclosure. *P<0.05, **P<0.01, ***P<0.001, ****P<0.0001 P<0.05 is considered statistically significant.

### DETAILED DESCRIPTIONOF THE PREFERRED EMBODIMENTS

The detailed description provided below in connection with the appended drawings is intended as a description of the present disclosure and is not intended to represent the only forms in which the present disclosure may be constructed or utilized.

### 1. Definitions

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. According to embodiments of the present disclosure, preferred isomers can be prepared by asymmetric syntheses. The invention additionally encompasses compounds described herein as individual isomers substantially free of other isomers, and alternatively, as mixtures of various isomers.

Unless otherwise indicated, the term "alkyl" means a straight chain, branched and/or cyclic ("cycloalkyl") hydrocarbon having from 1 to 20 *(e.g.,* 1 to 10, 1 to 9, 1 to 8, 1 to 7, 1 to 6, 1 to 5, 1 to 4, 1 to 3, 1 to 2, or 1) carbon atoms. Examples of alkyl groups include methyl, ethyl, propyl, isopropyl, n-butyl, t-butyl, isobutyl, 2-isopropyl-3-methyl butyl, pentyl, pentan-2-yl, hexyl, isohexyl, heptyl, heptan-2-yl, 4,4-dimethylpentyl, octyl, 2,2,4-trimethylpentyl, nonyl, decyl, undecyl and dodecyl. Cycloalkyl moieties may be monocyclic or multicyclic, and examples include cyclopropyl, cyclobutyl, cyclopentyl, and cyclohexyl. Unless otherwise specified, each instance of an alkyl group is independently optionally substituted, *i.e.,* unsubstituted (an "unsubstituted alkyl") or substituted (a "substituted alkyl") with one or more substituents. In certain examples, the alkyl group is unsubstituted C₁₋₁₀ alkyl; preferably, unsubstituted C₁₋₆ alkyl. In certain examples, the alkyl group is propyl. In other examples, the alkyl group is hexyl

"Alkenyl" refers to a radical of a straight-chain or branched hydrocarbon group having from 2 to 20 carbon atoms, one or more carbon-carbon double bonds, and no triple bonds ("C₂-₂₀ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (Cs), pentadienyl (Cs), hexenyl (C₆), and the like. Additional examples of alkenyl include heptenyl (C₇), octenyl (C₈), octatrienyl (C₈), and the like. Unless otherwise specified, each instance of an alkenyl group is independently optionally substituted, *i.e.,* unsubstituted (an "unsubstituted alkenyl") or substituted (a "substituted alkenyl") with one or more substituents. In certain examples, the alkenyl group is unsubstituted C₂₋₁₀ alkenyl. In certain examples, the alkenyl group is substituted C₂₋₁₀ alkenyl.

Alkynyl" refers to a radical of a straight-chain or branched hydrocarbon group having from 2 to 20 carbon atoms, one or more carbon-carbon triple bonds, and optionally one or more double bonds ("C₂₋₂₀ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (Cs), hexynyl (C₆), and the like. Additional examples of alkynyl include heptynyl (C₇), octynyl (C₈), and the like. Unless otherwise specified, each instance of an alkynyl group is independently optionally substituted, *i.e.,* unsubstituted (an "unsubstituted alkynyl") or substituted (a "substituted alkynyl") with one or more substituents. In certain examples, the alkynyl group is unsubstituted C₂₋₁₀ alkynyl. In certain examples, the alkynyl group is substituted C₂₋₁₀ alkynyl.

"Carbocyclyl," "carbocycle," or "carbocyclic" refers to a radical of a non-aromatic cyclic hydrocarbon group having from 3 to 10 ring carbon atoms ("C₃₋₁₀ carbocyclyl") and zero heteroatoms in the non-aromatic ring system. In some examples, a carbocyclyl group has 3 to 8 ring carbon atoms ("C₃₋₈ carbocyclyl"). In some examples, a carbocyclyl group has 3 to 6 ring carbon atoms ("C₃₋₆ carbocyclyl"). In some examples, a carbocyclyl group has 3 to 6 ring carbon atoms ("C₃₋₆ carbocyclyl"). In some examples, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (Cs), cyclopentenyl (Cs), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1H-indenyl (C₉), decahydronaphthalenyl (C₁₀), spiro[4.5]decanyl (C₁₀), and the like. As the foregoing examples illustrate, in certain examples, the carbocyclyl group is either monocyclic ("monocyclic carbocyclyl") or contain a fused, bridged. or spiro ring system such as a bicyclic system ("bicyclic carbocyclyl"). Carbocyclylcan be saturated, and saturated carbocyclyl is referred to as "cycloalkyl." In some examples, carbocyclyl is a monocyclic, saturated carbocyclyl group having from 3 to 10 ring carbon atoms ("C₃₋₁₀ cycloalkyl"). In some examples, a cycloalkyl group has 3 to 8 ring carbon atoms ("C₃₋₈ cycloalkyl"). In some examples, a cycloalkyl group has 3 to 6 ring carbon atoms ("C₃₋₆ cycloalkyl"). **In** some examples, a cycloalkyl group has 5 to 6 ring carbon atoms ("C₅₋₆ cycloalkyl"). **In** some examples, a cycloalkyl group has 5 to 10 ring carbon atoms ("C₅₋₁₀ cycloalkyl"). Examples of C₅₋₆ cycloalkyl groups include cyclopentyl (C₅) and cyclohexyl (Cs). Examples of C₃₋₆ cycloalkyl groups include the aforementioned C₅₋₆ cycloalkyl groups as well as cyclopropyl (C₃) and cyclobutyl (C₄). Examples of C₃₋₈ cycloalkyl groups include the aforementioned C₃₋₆ cycloalkyl groups as well as cycloheptyl (C₇) and cyclooctyl (Cs). Unless otherwise specified, each instance of a cycloalkyl group is independently unsubstituted (an "unsubstituted cycloalkyl") or substituted (a "substituted cycloalkyl") with one or more substituents. **In** certain examples, the cycloalkyl group is unsubstituted C₃₋₁₀ cycloalkyl. **In** certain examples, the cycloalkyl group is substituted C₃₋₁₀ cycloalkyl. Carbocyclyl can be partially unsaturated. Carbocyclyl including one or more C=C double bond in the carbocyclic ring is referred to as "cycloalkenyl." Carbocyclyl including one or more C≡C triple bond in the carbocyclic ring is referred to as "cycloalkynyl." Carbocyclyl includes aryl. "Carbocyclyl" also includes ring systems wherein the carbocyclic ring, as defined above, is fused with one or more aryl or heteroaryl groups wherein the point of attachment is on the carbocyclic ring, and in such instances, the number of carbons continue to designate the number of carbons in the carbocyclic ring system. Unless otherwise specified, each instance of a carbocyclyl group is independently optionally substituted, *i.e.,* unsubstituted (an "unsubstituted carbocyclyl") or substituted (a "substituted carbocyclyl") with one or more substituents. **In** certain examples, the carbocyclyl group is unsubstituted C₃₋₁₀ carbocyclyl. **In** certain examples, the carbocyclyl group is substituted C₃₋₁₀ carbocyclyl.

"Heterocyclyl," "heterocycle," or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). **In** heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged, or spiro ring system, such as a bicyclic system ("bicyclic heterocyclyl"), and can be saturated or can be partially unsaturated. Heterocyclyl bicyclic ring systems can include one or more heteroatoms in one or both rings. Heterocyclyl includes heteroaryl. Heterocyclyl also includes ring systems wherein the heterocyclic ring, as defined above, is fused with one or more carbocyclyl groups wherein the point of attachment is either on the carbocyclyl or heterocyclic ring, or ring systems wherein the heterocyclic ring, as defined above, is fused with one or more aryl or heteroaryl groups, wherein the point of attachment is on the heterocyclic ring, and in such instances, the number of ring members continue to designate the number of ring members in the heterocyclic ring system. Unless otherwise specified, each instance of heterocyclyl is independently optionally substituted, *i.e.,* unsubstituted (an "unsubstituted heterocyclyl") or substituted (a "substituted heterocyclyl") with one or more substituents. **In** certain examples, the heterocyclyl group is unsubstituted 3-10 membered heterocyclyl. **In** certain examples, the heterocyclyl group is substituted 3-10 membered heterocyclyl.

**In** some examples, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). **In** some examples, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). **In** some examples, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). **In** some examples, the 5-6 membered heterocyclyl has 1-3 ring heteroatoms selected from nitrogen, oxygen, and sulfur. **In** some examples, the 5-6 membered heterocyclyl has 1-2 ring heteroatoms selected from nitrogen, oxygen, and sulfur. **In** some examples, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Aryl" refers to a radical of a monocyclic or polycyclic (e.g., bicyclic or tricyclic) 4n+2 aromatic ring system (e.g., having 6, 10, or 14 pi electrons shared in a cyclic array) having 6-14 ring carbon atoms and zero heteroatoms provided in the aromatic ring system ("C₆₋₁₄ aryl"). In some examples, an aryl group has six ring carbon atoms ("C₆ aryl"; *e.g.,* phenyl). In some examples, an aryl group has ten ring carbon atoms ("C₁₀ aryl"; e.g., naphthyl such as 1-naphthyl and 2-naphthyl). In some examples, an aryl group has fourteen ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e.,* unsubstituted (an "unsubstituted aryl") or substituted (a "substituted aryl") with one or more substituents. In certain examples, the aryl group is unsubstituted C₆₋₁₄ aryl. In certain examples, the aryl group is substituted C₆₋₁₄ aryl.

"Aralkyl" is a subset of alkyl and aryl, as defined herein, and refers to an optionally substituted alkyl group substituted by an optionally substituted aryl group. In certain examples, the aralkyl is optionally substituted benzyl. In certain examples, the aralkyl is benzyl. In certain examples, the aralkyl is optionally substituted phenethyl. In certain examples, the aralkyl is phenethyl.

"Aralkenyl" is a subset of alkenyl and aryl, as defined herein, and refers to an optionally substituted alkenyl group substituted by an optionally substituted aryl group. An example of aralkenyl is styrenyl *(i.e.,* -CH=CHPh).

"Aralkynyl" is a subset of alkynyl and aryl, as defined herein, and refers to an optionally substituted alkynyl group substituted by an optionally substituted aryl group.

"Heteroaryl" refers to a radical of a 5-10 membered monocyclic or bicyclic 4n+2 aromatic ring system (e.g., having 6 or 10 pi electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). **In** heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, *i.e.,* either the ring bearing a heteroatom *(e.g.,* 2-indolyl) or the ring that does not contain a heteroatom (e.g., 5-indolyl).

**In** some examples, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). **In** some examples, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). **In** some examples, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). **In** some examples, the 5-6 membered heteroaryl has 1-3 ring heteroatoms selected from nitrogen, oxygen, and sulfur. **In** some examples, the 5-6 membered heteroaryl has 1-2 ring heteroatoms selected from nitrogen, oxygen, and sulfur. **In** some examples, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. **In** certain examples, the heteroaryl group is unsubstituted 5-14 membered heteroaryl. **In** certain examples, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Heteroaralkyl" is a subset of alkyl and heteroaryl, as defined herein, and refers to an optionally substituted alkyl group substituted by an optionally substituted heteroaryl group.

"Heteroaralkenyl" is a subset of alkenyl and heteroaryl, as defined herein, and refers to an optionally substituted alkenyl group substituted by an optionally substituted heteroaryl group.

"Heteroaralkynyl" is a subset of alkynyl and heteroaryl, as defined herein, and refers to an optionally substituted alkynyl group substituted by an optionally substituted heteroaryl group.

The term "alkoxyl" refers to a moiety for the formula: -OR', wherein R' is an optionally substituted alkyl described above.

The term "substituted," when used to describe a chemical structure or moiety, refers to a derivative of that structure or moiety wherein one or more of its hydrogen atoms is substituted with one or more of: alkyl, halo, haloalkyl, hydroxyl, amino, alkylamino, or dialkylamino.

An atom, moiety, or group described herein may be unsubstituted or substituted, as valency permits, unless otherwise provided expressly. The term "optionally substituted" refers to substituted or unsubstituted.

In general, the term "substituted", whether preceded by the term "optionally" or not, means that at least one hydrogen present on a group *(e.g.,* a carbon or nitrogen atom) is replaced with a permissible substituent, e.g., a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety. In certain examples, the substituent is a carbon atom substituent. In certain examples, the substituent is a nitrogen atom substituent. In certain examples, the substituent is an oxygen atom substituent.

It should also be noted that if the stereochemistry of a structure or a portion of a structure is not indicated with, for example, bold or dashed lines, the structure or the portion of the structure is to be interpreted as encompassing all stereoisomers of it. Similarly, names of compounds having one or more chiral centers that do not specify the stereochemistry of those centers encompass pure stereoisomers and mixtures thereof. Specific enantiomers can be separated and collected by the techniques known in the art such as chromatography in chiral stationary phase or chiral salt formation followed by separation based on selective crystallization. By using a specific enantiomer as a starting substance, it is also possible to obtain a corresponding isomer as the final product.

Unless otherwise indicated, any atom shown in a drawing with unsatisfied valences is assumed to be attached to enough hydrogen atoms to satisfy the valences.

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid or by using other methods known in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄ alkyl)₄⁻ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

The term "solvate" refers to forms of the compound that are associated with a solvent, usually by a solvolysis reaction. This physical association may include hydrogen bonding. Conventional solvents include water, methanol, ethanol, acetic acid, DMSO, THF, diethyl ether, and the like. The compounds described herein may be prepared, *e.g.,* in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solution-phase and isolatable solvates. Representative solvates include hydrates, ethanolates, and methanolates.

The term "administered", "administering" or "administration" are used interchangeably herein to refer a mode of delivery, including, without limitation, intraveneously, intramuscularly, intraperitoneally, intraarterially, intracranially, or subcutaneously administering an agent (e.g., a compound or a composition) of the present invention. In some examples, the compound of the present disclosure or a salt, a solvate thereof is formulated into tablets for oral administration. In other examples, t the compound of the present disclosure or a salt, a solvate thereof is formulated into powders for mixed with suitable carrier (e.g., buffer solution) before use, such as intraveneous injection.

The term "an effective amount" as used herein refers to an amount effective, at dosages, and for periods of time necessary, to achieve the desired result with respect to the treatment of a disease. For example, in the treatment of cancer, an agent (i.e., the present compound) which decrease, prevents, delays or suppresses or arrests any symptoms of the cancer would be effective. An effective amount of an agent is not required to cure a disease or condition but will provide a treatment for a disease or condition such that the onset of the disease or condition is delayed, hindered or prevented, or the disease or condition symptoms are ameliorated. The effective amount may be divided into one, two or more doses in a suitable form to be administered at one, two or more times throughout a designated time period.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The singular forms "a", "and", and "the" are used herein to include plural referents unless the context clearly dictates otherwise.

### 2. The compound of the present invention

Described herein are polyhydroxyated indolizidine and pyrrolizidine derivatives having the structures as set forth herein, and uses of one or more of such compounds for treating cancers.

Accordingly, it is the first aspect of the present disclosure to provide a compound capable of selectively inhibiting the activity of α-hGMII over that of α-hLM. The compound has the structure of formula (I), 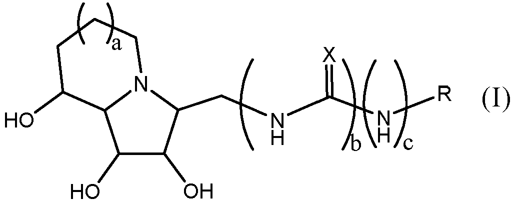 a salt, or a solvate thereof.

In formula (I), X is O or S; a, b and c are independently an integral of 0 or 1; and R is selected from the group consisting of H, C₁₋₆ alkyl, alkenyl, alkynyl, aryl, heteroaryl, cycloalkyl, cycloalkenyl, aralkyl, aralkenyl, aralkynyl, heteroaralkyl, heteroaralkenyl, heteroaralkynyl, heterocyclyl, alkoxy, aryloxy, and sulfonyl.

The compound may have the structure of formula (II),

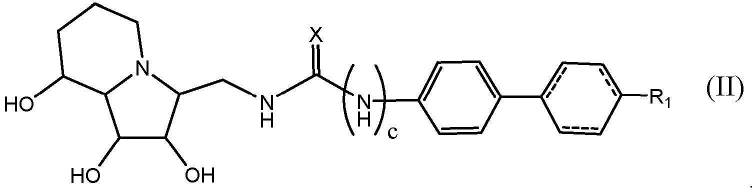

In formula (II), ------ is a single or double bond; X is O or S; c is 0 or 1; and R₁ is hydrogen or C₁₋₆ alkyl.

The compound of formula (II) is selected from the group consisting of:

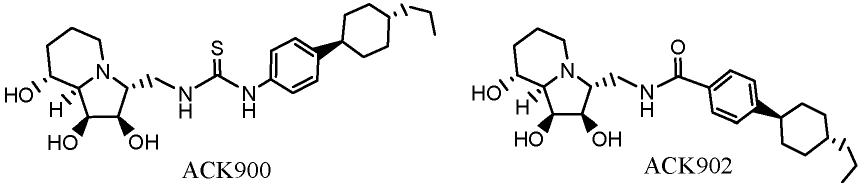

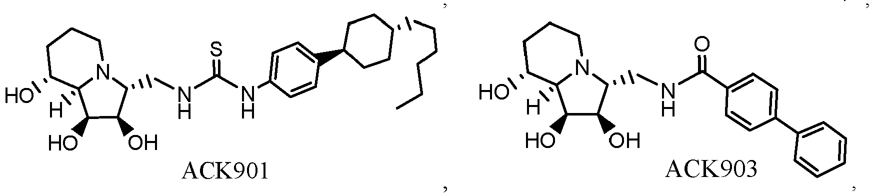

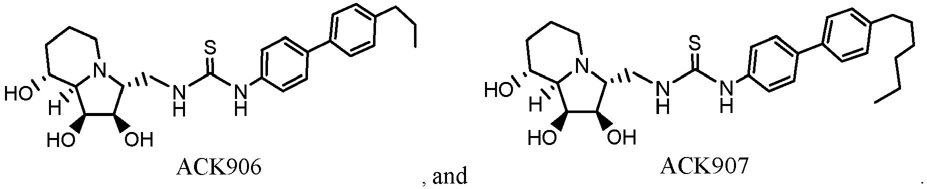

The compound may have the structure of formula (III),

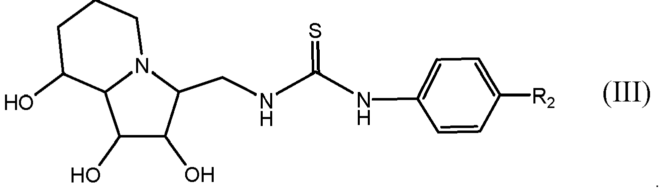

In formula (III), R₂ is hydrogen, C₁₋₆ alkyl, or alkoxy

The compound of formula (III) can be any one of,

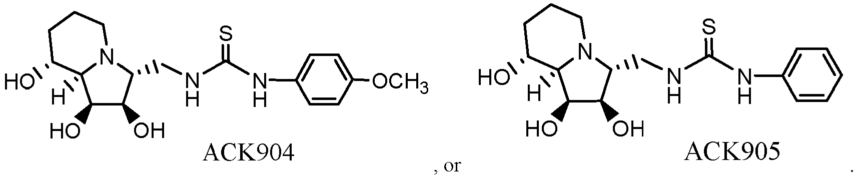

Thehe compound may have the structure of formula (IV), 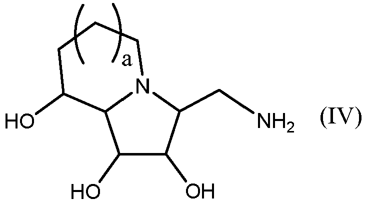 wherein:
a is 0 or 1.

In formula (IV), a is 0 or 1.

According to the present disclosure, any one of the compounds of formula (I) to (IV) has a selective inhibitory activity toward α-hGMII than α-hLM. Preferably, any one of the compounds of formula (I) to (IV) has a selective index (SI) in the range of 3 to 150, such as 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150; more preferably, any one of the compounds of formula (I) to (IV) has a selective index (SI) in the range of 20 to 140, such as 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, and 140. In certain examples, the compound of formula (II) (e.g., ACK902) has an SI of 4.3, i.e., the compound of formula (II) suppresses α-hGMII at an IC₅₀ dose at least 4.3 folds lower than that of α-hLM. In other examples, the compound of formula (II) (e.g., ACK902) has an SI of 21.5, i.e., the compound of formula (II) suppresses α-hGMII at an IC₅₀ dose at least 21.5 folds lower than that of α-hLM. In further examples, the compound of formula (II) (e.g., ACK902) has an SI of 140, i.e., the compound of formula (II) suppresses α-hGMII at an IC₅₀ dose at least 140 folds lower than that of α-hLM.

It is the second aspect of the present disclosure to provide a pharmaceutical composition, which comprises a compound of any one of formula (I) to (IV), or a pharmaceutically acceptable salt thereof, and a pharmaceutically acceptable excipient

It is the third aspect of the present disclosure to provide the compounds disclosed herein for use in treating a subject having a cancer by administering to the subject an effective amount of any one of compounds of formula (I), (II), (III) or (IV) described above.

Examples of cancer that may be treated include, but are not limited to, cancer is selected from the group consisting of bone tumor, brain cancer, breast cancer, cervical cancer, CNS neoplasm, colon cancer, esophageal cancer, Ewing's sarcoma, head and neck cancer, Hodgkin's disease, larynx cancer, leukemia, liver cancer, lymphoma, melanoma, multiple myeloma, nasopharynx cancer, neuroblastoma, non-small-cell lung (NSCL) cancer, pancreatic cancer, prostate cancer, rectal cancer, retinoblastoma, small-cell lung (SCL) cancer, testicular cancer, thyroid cancer, skin cancer other than melanoma, and Wilms' tumor.

In some embodiments of the present disclosure, the cancer is a metastatic cancer.

In all embodiments, the subject is a human

The compound of the present invention may be independently produced by methods set forth in the working examples or via any methods known in the related art. Each compound thus produced is subject to bioactivity analysis to see if it possessed selective inhibitory activity towards α-hGMII, which leads to the suppression of the growth, migratory and invasion of cancer cells, accordingly, the compound may serve as a candidate for the development of medicaments suitable for treating cancers.

### 3. Pharmaceutical compositions and kits

Another aspect of the present disclosure relates to pharmaceutical compositions comprising one or more of the compounds as described herein (e.g., Compounds ACK900, ACK901, and ACK 902), and optionally a pharmaceutically acceptable excipient.

Any of the pharmaceutical compositions described herein can be formulated for a suitable administration route, e.g., orally, parenterally, by inhalation spray, topically, rectally, nasally, buccally, vaginally or via an implanted reservoir. The term "parenteral" as includes subcutaneous, intracutaneous, intravenous, intramuscular, intraarticular, intraarterial, intrasynovial, intrasternal, intrathecal, intralesional and intracranial injection or infusion techniques.

A sterile injectable pharmaceutical composition, e.g., a sterile injectable aqueous or oleaginous suspension, can be formulated according to techniques known in the art using suitable dispersing or wetting agents (such as Tween^{®} 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of inj ectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purposes of formulation.

A pharmaceutical composition for oral administration can be any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added. A nasal aerosol or inhalation pharmaceutical composition can be prepared according to techniques well-known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art. A pharmaceutical composition of the invention can also be administered in the form of suppositories for rectal administration.

Pharmaceutically acceptable excipients that may be included in a pharmaceutical composition of the invention include inert diluents, solubilizing agents, dispersing and/or granulating agents, surface active agents and/or emulsifiers, disintegrating agents, binding agents, preservatives, buffering agents, lubricating agents, and/or oils. Excipients such as cocoa butter and suppository waxes, coloring agents, coating agents, sweetening, flavoring, and perfuming agents may also be present in the pharmaceutical composition.

An excipient present in an inventive pharmaceutical composition must be "pharmaceutically acceptable" in the sense that the excipient is compatible with the active ingredient of the pharmaceutical composition (and preferably, capable of stabilizing the pharmaceutical composition) and not deleterious to a subject to whom the pharmaceutical composition is administered. For example, solubilizing agents such as cyclodextrins, which may form specific, more soluble complexes with the compounds of the invention, can be utilized as pharmaceutically acceptable excipients for delivery of the compounds of the invention into the subject. Examples of other pharmaceutically acceptable excipients include colloidal silicon dioxide, magnesium stearate, cellulose, sodium lauryl sulfate, and D&C Yellow # 10.

Also disclosed herein are kits (e.g., pharmaceutical packs) comprising one or more of the compounds or pharmaceutical compositions described herein, and a container (e.g., a vial, ampule, bottle, syringe, and/or dispenser package, or other suitable container). In some examples, the kits may include a second container comprising a pharmaceutically acceptable excipient for dilution or suspension of an inventive pharmaceutical composition or compound. In some examples, the inventive pharmaceutical composition or compound provided in the first container and the second container are combined to form one unit dosage form.

In certain examples, a kit as described herein is for use in inhibiting the activity of α-hGMII over α-hLM, or all of them, in cells. In certain examples, a kit as described herein is for use in treating any of the target diseases as described herein, e.g., cancer in a subject in need thereof, or inhibiting cancer cells.

Any of the kits described herein can thus include instructions for administering the compound or pharmaceutical composition contained therein. A kit of the invention may also include information as required by a regulatory agency such as the FDA. In certain examples, the information included in the kit is prescribing information. In certain examples, the kit and instructions provide for selectively inhibiting the activity of α-hGMII than α-hLM. In certain examples, the kit and instructions provide for inhibiting the activity of α-hGMII. In certain examples, the kit and instructions provide for treating a disease described herein. In certain examples, the kit and instructions provide for preventing a disease described herein. A kit of the invention may include one or more additional pharmaceutical agents described herein as a separate composition.

### 4. Use in treatment

Any of the compounds or pharmaceutical compositions described herein can be used for selectively inhibiting the activity of α-hGMII. They also can be used in treating a disease associated with α-hGMII, or an EGFR, including, but are not limited to, cancers.

In some examples, the use in treatment described herein can comprise administering to a subject in need of the treatment an effective amount of the pharmaceutical composition as described herein. The term "treating" or "treatment" as used herein refers to the application or administration of a pharmaceutical composition or compound as described herein to a subject, who has a disorder (e.g., cancer), a symptom of the disorder, a disease or disorder secondary to the disorder, or a predisposition toward the disorder, with the purpose to cure, alleviate, relieve, remedy, or ameliorate the disorder, the symptom of the disorder, the disease or disorder secondary to the disorder, or the predisposition toward the disorder.

A "subject" to be treated by administering the compounds described herein can be a human subject *(e.g.,* a pediatric subject such as an infant, a child, or an adolescent, or an adult subject such as a young adult, middle-aged adult, or senior adult), or a non-human animal, such as dogs, cats, cows, pigs, horses, sheep, goats, rodents (e.g., mice, rats), and non-human primates (e.g., cynomolgus monkeys, rhesus monkeys). The non-human mammal may be a transgenic animal or genetically engineered animal. In some examples, the subject is a human patient having a target disease as described herein (e.g., cancer), suspected of having the disease, or is at risk for the disease.

In some examples, the subject is a human or non-human mammal having, suspected of having, or at risk for cancer. The term "cancer" refers to a class of diseases characterized by the development of abnormal cells that proliferate uncontrollably and have the ability to infiltrate and destroy normal body tissues. The compounds described herein are useful in treating cancers of any type, particularly those that are responsive to inhibition of α-hGMII activity. Examples include, but are not limited to, bone tumor, brain cancer, breast cancer, cervical cancer, CNS neoplasm, colon cancer, esophageal cancer, Ewing's sarcoma, head and neck cancer, Hodgkin's disease, larynx cancer, leukemia, liver cancer, lymphoma, melanoma, multiple myeloma, nasopharynx cancer, neuroblastoma, non-small-cell lung (NSCL) cancer, pancreatic cancer, prostate cancer, rectal cancer, retinoblastoma, small-cell lung (SCL) cancer, testicular cancer, thyroid cancer, skin cancer other than melanoma, and Wilms' tumor. **In** other examples, the subject is a human patient having metastatic cancer.

An "effective amount" of a compound described herein (either taken alone or in combination of another agent) refers to an amount sufficient to elicit the desired biological response, e.g., selectively inhibiting α-hGMII activity, or alleviating a target disease described herein or a symptom associated with the disease. As will be appreciated by those of ordinary skill in this art, the effective amount of a compound described herein may vary depending on such factors as the desired biological endpoint, the pharmacokinetics of the compound, the condition being treated, the mode of administration, and the age and health of a subject. **In** some examples, an effective amount can be a therapeutically effective amount, which refers to an amount of a therapeutic agent, alone or in combination with other therapies, sufficient to provide a therapeutic benefit in the treatment of a condition or to delay the onset or minimize one or more symptoms associated with the condition. The term "therapeutically effective amount" can encompass an amount that improves overall therapy, reduces or avoids symptoms, signs, or causes of the condition, and/or enhances the therapeutic efficacy of another therapeutic agent. **In** other examples, the effective amount can be a prophylactically effective amount. A prophylactically effective amount of a compound means an amount of a therapeutic agent, alone or in combination with other agents, which provides a prophylactic benefit in the prevention of the condition. For example, a "prophylactically effective amount" of a compound can be an amount sufficient to prevent or delay the onset of a condition, or one or more symptoms associated with the condition or prevent its recurrence. It may also be an amount that improves overall prophylaxis or enhances the prophylactic efficacy of another prophylactic agent.

**In** some examples, use of the compound described herein is performed by administering one or more compounds of formula (I) to (IV) or pharmaceutical compositions described herein to a subject in need of the treatment (e.g., any of the subject described herein such as a human cancer patient) in an amount effective in selectively inhibiting α-hGMII activity in the subject.

According to the present disclosure, any one of the compounds of formula (I) to (IV) has a selective inhibitory activity toward Golgi α-hGMII than α-hLM. Preferably, any one of the compounds of formula (I) to (IV) has a selective index (SI) in the range of 3 to 150, such as 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, and 150; more preferably, any one of the compounds of formula (I) to (IV) has a selective index (SI) in the range of 20 to 140, such as 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, and 140. In certain examples, the compound of formula (II) (e.g., ACK902) has an SI of 4.3, i.e., the compound of formula (II) suppresses α-hGMII at an IC₅₀ dose at least 4.3 folds lower than that of α-hLM. In other examples, the compound of formula (II) (e.g., ACK902) has an SI of 21.5, i.e., the compound of formula (II) suppresses α-hGMII at an IC₅₀ dose at least 21.5 folds lower than that of α-hLM. In further examples, the compound of formula (II) (e.g., ACK902) has an SI of 140, i.e., the compound of formula (II) suppresses α-hGMII at an IC₅₀ dose at least 140 folds lower than that of α-hLM.

In other examples, the compound(s) or pharmaceutical composition(s) is administered to the subject in an amount effective in treating a target disease as described herein, e.g., cancer.

An effective amount of a compound may vary from about 0.01 mg/kg to about 1,000 mg/kg in one or more dose administrations for one or several days (depending on the mode of administration). In certain examples, the effective amount per dose varies from about 0.01 mg/kg to about 1,000 mg/kg, from about 0.1 mg/kg to about 750 mg/kg, from about 0.1 mg/kg to about 500 mg/kg, from about 1.0 mg/kg to about 250 mg/kg, and from about 10.0 mg/kg to about 150 mg/kg.

The terms "administer," "administering," or "administration" refer to implanting, absorbing, ingesting, injecting, inhaling, or otherwise introducing a compound or pharmaceutical composition of the invention, in or on a subject. Any of the suitable administration routes can be used for delivering the compounds or pharmaceutical compositions described herein. Examples include, but are not limited to, orally, parenterally, by inhalation spray, topically, rectally, nasally, buccally, vaginally or via an implanted reservoir.

Also described herein are ways for selectively inhibiting the activity of α-hGMII than α-hLM in cells, which may comprise contacting one or more compounds as described herein with cells in an amount effective to inhibit the activity of α-hGMII. The amount of the one or more compounds can be sufficient to inhibit at least 20% (e.g., 30%, 40%, 50%, 60%, 70%, 80%, 90%, or 95%) of the enzymatic activity. In some examples, this can be performed *in intro.* In other examples, they can be performed *in vivo* by administering the compound to a subject in need of the treatment as described herein.

It will be also appreciated that a compound or pharmaceutical composition, as described herein, can be used in combination with one or more additional pharmaceutical agents (e.g., therapeutically and/or prophylactically active agents) in any of the uses described herein. The compounds or pharmaceutical compositions can be administered in combination with additional pharmaceutical agents that improve their activity (e.g., activity (e.g., potency and/or efficacy) in treating a disease described herein in a subject in need thereof, in preventing a disease described herein in a subject in need thereof, in selectively inhibiting the activity of α-hGMII than α-hLM in a subject or cell, in selectively inhibiting the activity of α-hGMII in a subject or cell, in a subject or cell, bioavailability, and/or safety, reduce drug resistance, reduce and/or modify their metabolism, inhibit their excretion, and/or modify their distribution within the body of a subject. It will also be appreciated that the therapy employed may achieve a desired effect for the same disorder, and/or it may achieve different effects. In certain examples, an inventive pharmaceutical composition including a compound of the invention and an additional pharmaceutical agent shows a synergistic effect that is absent in a pharmaceutical composition including one of the compound and the additional pharmaceutical agent, but not both.

In some examples, the compound or pharmaceutical composition can be administered concurrently with, prior to, or subsequent to one or more additional pharmaceutical agents, which may be useful as, e.g., combination therapies. Pharmaceutical agents can be therapeutically active agents or prophylactically active agents. The additional pharmaceutical agent includes, but is not limited to, anti-cancer agent. In certain examples, the additional pharmaceutical agent is an α-hGMII inhibitor. In certain examples, the compounds or pharmaceutical compositions described herein can be administered in combination with an anti-cancer therapy including, but not limited to, surgery, radiation therapy, and chemotherapy.

The compounds described herein are capable of reducing cancer cell migration and/or invasion, one or more compounds as described herein can be co-used with such anti-migration drugs so as to improve their therapeutic efficacy. In some examples, the anti-migration drug for co-use with any of the compounds described herein (e.g., the compound described in Example 1 below) can be a platinum-based antineoplastic agent, e.g., oxaliplatin, cisplatin, carboplatin, satraplatin, picoplatin, nedaplatin, or triplatin.

Another aspect of the invention relates to methods of screening a library of compounds, and pharmaceutical acceptable salts thereof, to identify a compound, or a pharmaceutical acceptable salt thereof, which is useful in the invention. In certain examples, the methods of screening a library include obtaining at least two different compounds of the invention; and performing an assay using the different compounds of the invention. In certain examples, the assay is useful in identifying a compound that is useful in the inventive use.

The present invention will now be described more specifically with reference to the following examples, which are provided for the purpose of demonstration rather than limitation. While they are typically of those that might be used, other procedures, methodologies, or techniques known to those skilled in the art may alternatively be used.

### EXAMPLES

### Materials and Methods

### Cell culture.

Human liver cancer HepG2 cells and Huh7 cells, breast cancer MDA-MB-231 cells, pancreatic cancer PANC-1 cells, and non-small cell lung (NSCL) cancer H1975 cells were maintained in Dulbecco's Modified Eagle Medium supplemented containing 10% fetal bovine serum (FBS), 100 units/ml penicillin and 100 mg/ml streptomycin in 37°C incubator with 5% CO₂.

### Golgi α-mannosidase II (α-hGMII) and α-mannosidase (α-hLM) activity assay

Compounds were diluted to give the final concentration of 100 µM and mixed with 4-MU-α-D-mannopyranoside as substrate and human Golgi α-mannosidase II in phosphate buffer (0.1 M Sodium phosphate dibasic, pH 7.0) or human lysosomal α-mannosidase in citric phosphate buffer (0.1 M Sodium phosphate monobasic monohydrate, 0.5 mM citric acid monohydrate, pH 4.6). The assay was carried out at 37°C for a certain time (1 h for α-hLM, 2 h for α-hGMII). Stop solution (0.5 M K₂CO_{3(aq)}, pH 10.8) was then added to the reactions and the fluorescence was determined at 355 nm excitation and 460 nm emission (SpectraMax M5, Molecular Devices). Inhibition was performed as relative enzyme activity to control. The active compounds were selected for libraries preparation and further tested at lower concentration to determine their IC₅₀ values. The assays performed in 384-wells of the microtiter plates.

### Anti-proliferative activity assay

Anti-proliferative activity of HepG2, Huh7 or PANC-1 cells was determined by 3-(4,5-dimethylthiazol-2-yl)-2,5-diphenyltetrazolium bromide (MTT) assay, while that of H1975 or MDA-MB-231 cells was determined by luminescent assay with the aid of commercial kit - CellTiter-Glo^{®} (Promega).

Cancer cells including HepG2, Huh7 and PANC-1 cells were respectively seeded at 10⁴, 4x10³, and 5x10³ cells/well in 96-well plates and maintained for 14-16 hours. Cells were treated with dimethylsulfoxide (DMSO) or test compounds in varied concentrations for 72 h. Cells were then washed with PBS twice, added a medium containing 3-(4,5-dimethylthiazol-2-yl)-2,5-diphenyltetrazolium bromide (MTT) at a final concentration of 0.5 mg/mL, and incubated for 4 h at 37°C in a humidified incubator containing 5% CO₂ in air. Cells having functional succinate dehydrogenase of mithchondria would convert MTT to formazan. Then, the medium was replaced with 100 µL of DMSO for 30 min at room temperature, and the 96-well plate was read by an ELISA reader at 550 nm to get the absorbance density values. The IC₅₀ values of death cell lines were calculated accordingly.

For H1975 and MDA-MB-231, cells were respectively seeded at 5x10³ cells/well in 96-well plates and maintained for 14-16 hours. Cells were treated with dimethylsulfoxide (DMSO) or test compounds in varied concentrations for 48 h. Cell viability was monitored using Cell-TiterGlo (Promega), a kit that measures the quantity of ATP present in the cell culture, an indicator of metabolically active cells. The IC₅₀ values of death cell lines were calculated accordingly.

### Transwell Migration Assay (chemokines and chemotaxis assay)

Chemotaxis experiments with human HepG2, Huh7, or PANC-1 cells were assayed by using transwells (Millipore). The cells to be analyzed (5 × 10⁴ cells/well) were seeded in the upper chambers of the 24-well plate in 400 µL serum-free medium. FBS (10%) was used as the chemoattractant with or without different concentrations of the test compound (e.g., **ACK900, ACK901, ACK902)** in the lower chamber, and the plate was incubated under standard conditions. After incubation for 18 hours, the media in the upper chambers were aspirated and washed with phosphate-buffered saline (PBS) twice. Both non-migrated and migrated cells were fixed with 4% paraformaldehyde (PFA) for 30 minutes at room temperature, stained with 2% crystal violet solution. The non-invading cells were removed from the upper surface of the insert membrane with a cotton swab. The images were taken using an imaging reader (Cytation 5, BioTek, VT, U.S.A.) and analyzed using NIH ImageJ software. Percent in the results represent percent of initial number of cells in the migrating and nonmigrating cell fractions.

### Serum biochemistry assay

All blood specimens were collected in tubes and were left at 25 °C for 30 min to clot and centrifuged for 10 min at 1200 g. The concentrations of the following biochemical serum analytes collected were measured with FUJI DRI-CHEM 500i analyzer (FUJIFILM Corp., Tokyo, Japan) using the manufacturer's reagents and according to the manufacturer's instructions: Alanine Aminotransferase (ALT), Aspartate Aminotransferase (AST), Blood Urea Nitrogen (BUN), Creatinine (CRE) and Total Bilirubin (TBIL).

### Animals.

A total of 34 NOD-SCID mice (5- to 6-week-old) were maintained in micro-isolator units on a standard laboratory diet. Animals were housed under humidity- and temperature-controlled conditions and the light/dark cycle was set at 12-hour intervals, maintained under specified and opportunistic pathogen-free conditions. Mice were quarantined at least 1 week before experimental manipulation.

### Human hepatocarcinoma (HCC) Huh7cells xenograft animal model

HCC xenografts were established in 5-6 weeks old NOD-SCID mice by subcutaneous inoculation of about 5 x 10⁶ Huh7 cells suspended in 50 µL PBS + 50 µL Matrigel (Corning, 394230) in the left and right flanks of each mouse. After one week of inoculation, tumors were palpable and the mice were assigned randomly to 1 of 4 treatment groups. Group 1 (vehicle control group, n=6) mice only received a vehicle solution containing Kolliphor EL (Sigma Aldrich, C5135), 99.9% ethanol and water in a ratio of 1:1:6. Groups 2, 3, 4 and 5 mice were intraperitoneally injected with the test compound (e.g., compound **ACK900, ACK901, or ACK902)** (30 mg/kg, ip, n=7/group) or sorafenib (30 mg/kg, ip, n=6/group), twice per week for 25 days. The tumor volume was calculated by the formula of a ratioinal ellipsoid: [0.5236 x (short axis) m₁² x (long axis) m²], and the tumor volume was measured twice per week. All mice were sacrificed at 27 days after the treatment started. Tumor tissues were isolated, photographed and weighted.

### Example 1 Synthesis of the compound of the present invention

The compounds of the present invention were synthesized in accordance with **schemes 1 and 2.**

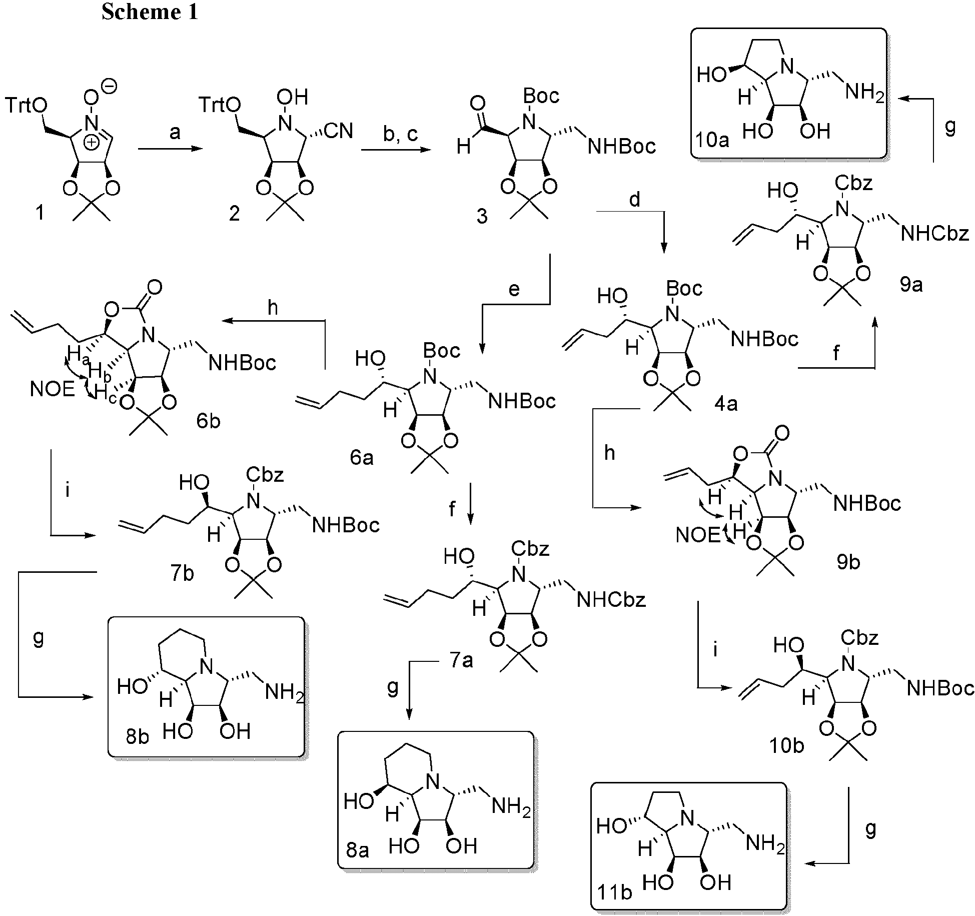

Reagents and conditions: Reagents and conditions: (a) TMSCN, MeOH, 50 °C, 2 h, 90%. (b) (1) Raney Ni, H₂, Boc₂O, MeOH, 8 h, rt, 74%; (2) HCOOH, Et₂O, rt, 2 h, 78%. (c) DMP, CH₂Cl₂, rt, 2 h, 99%. (d) AllylMgCl, THF, -78 °C, 1 h, **4a:epi-4a** = 3.1:1, total yield 84%. (e) 3-butenylMgBr, THF, 0 °C to rt, 1 h, 78%. (f) (1) ZnBr₂, CH₂Cl₂, rt, 12 h; (2) CbzCl, NaHCO_{3(aq)}, THF, rt, 3 h, 76% **(5a)** and 69% **(8a)** over 2 steps. (g) (1) OsO₄, NaIO₄, 2,6-lutidine, dioxane/H₂O, rt, 8 h; (2) Pd/C, H₂, MeOH, rt, o.n. (3) HCl, MeOH, rt, 8 h, 84% **(6a),** 78% **(6b),** 80% **(9a)** and 90% **(9b)** over 3 steps. (h) Tf₂O, py, CH₂Cl₂, 0 °C to rt, 3 h, 75% **(4b)** and 73% (7**b**). (i) (1) LiOH, EtOH, H₂O, 90 °C, 12 h; (2) CbzCl, NaHCO_{3(aq)}, THF, rt, 3 h, 73% **(5b)** and 69% **(8b)** over 2 steps.

The synthetic journey toward the target scaffolds **(6a, 6b, 9a** and **9b)** commenced with cyclic nitrone 1 via highly nucleophilic addition of trimethylsilyl cyanide (TMSCN), followed by cleavage of N-O bond and reduction of nitrile moiety in the presence of Boc₂O (Raney Ni/H₂), selective deprotection of trityl group and Dess-Martin periodinane (DMP) oxidation, bis-N-Boc protected aldehyde **3** was furnished smoothly in multigram scale. The corresponding aldehyde **3** was proceeded to Allyl Grignard addition at -78 °C to smoothly yield two separable epimeric homoallylic alcohol in a ratio of 3.1:1, slightly favoring the Felkin-Ahn stereoisomer **4a.** This observation was consonant with the results published by us. The major product **4a** (less polar) was isolated by column chromatography and the stereochemistry of newly generated chiral center in homoallylic alcohol 4a was determined precisely by X-ray crystallographic analysis.

With homoallylic alcohol **4a** in hand, two *N-Boc* groups of **4a** was switched from *N-*terminus Boc to Cbz by treatment with ZnBr₂ in CH₂Cl₂ followed by Cbz protection obtained bis-N-Cbz alkenyl pyrrolidine **5a** in 76% yield. To obtained terminal aldehyde, OsO₄-NaIO₄ mediated oxidative cleavage was used and finally, the resulting aldehyde was treated with neutral hydrogenated reductive amination with Pd/C and acetonide cleavage in acidic conditions to furnish the desired scaffold **6a** smoothly in excellent yield (84% over 3 steps). The configuration of **6a** was determined by 2D NOESY analysis and the newly generated stereogenic center in **4a** was re-analyzed.

For the synthesis of pyrrolizidine scaffold **6b,** which requires bis-*N*-Boc analogue of pyrrolidine **5b** with exactly opposite stereochemistry to that of alcohol **4a.** Configurational inversion was performed by treatment with triflic anhydride (Tf₂O) to generate cyclic carbamate **4b** with desired inverted chiral center in 73% yield. The stereochemistry of carbamate **4b** was determined by 2D NOESY NMR analysis showing that Hₐ, H_{b} and H_{c} have NOE observed. The cyclic carbamate **4b** was further treated with LiOH-mediated hydrolysis in refluxing ethanol and the resulting amino-alcohol was then subsequently subjected to Cbz protection to provide the *N-Cbz, N*-Boc-pyrrolidine **5b** in 69% over 2 steps. Following the cyclization method and acidic deprotection for **6a,** the target compound, **6b** was synthesized in 78% over 3 steps.

Having synthesized pyrrolizidine scaffolds **6a** and **6b,** the other two indolizidine scaffolds **9a** and **9b** were then prepared. To this end, homoallyl Grignard addition of aldehyde **3** from 0 °C to room temperature stereoselectively generated alkenyl pyrrolidine **7a** as single isomer. Compound **7a** was subjected to similar condition as that for **4a,** other two desired scaffolds **9a** and **9b** were obtained in a yield of 55% over 5 steps and 45% over 6 steps, respectively.

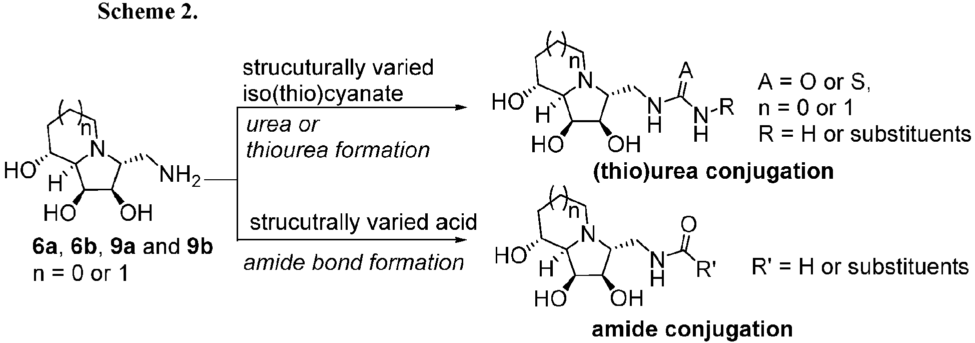

### 1.1 ACK900

1-(trans-4-propylcyclohexyl)-4-isothiocyanatobenzene (14 mg, 0.055 mmol) was added to a solution of **9b** (10 mg, 0.05 mmol) and Et₃N (14 µL, 0.1 mmol) in DMSO (0.5 mL) and stirred for 1 h. After the reaction was complete, the reaction was extracted with EtOAc and H₂O. The combined organic layers were dried (MgSO₄), filtered and concentrated. The residue was purified by column chromatography (CH₂Cl₂ / MeOH = 20:1, silica gel) to give **ACK900** (12 mg, 0.03 mmol, 52%) as a white solid.

### 1.2 ACK901

A mixture of **9b** (10 mg, 0.05 mmol) and Et₃N (14 µL, 0.1 mmol) in DMSO (0.5 mL) was added 1-(trans-4-hexylcyclohexyl)-4-isothiocyanatobenzene (16.6 mg, 0.055 mmol) and stirred for 1 h. The reaction extracted with EtOAc and H₂O. The combined organic layers were dried (MgSO₄), filtered and concentrated. The residue was purified by column chromatography (CH₂Cl₂ / MeOH = 20: 1, silica gel) to give **ACK901** (7 mg, 0.012 mmol, 60%) as a white solid.

### 1.3 ACK902

2,5-dioxopyrrolidin-1-yl 4-((1s,4r)-4-propylcyclohexyl) benzoate (52 mg, 0.15 mmol) was added to a solution of **9b** (10 mg, 0.05 mmol) and Et₃N (14 µL, 0.1 mmol) in DMSO (0.5 mL) and stirred for 8 h. After the reaction was complete, the reaction was extracted with EtOAc and H₂O. The combined organic layers were dried (MgSO₄), filtered and concentrated. The residue was purified by column chromatography (CH₂Cl₂ / MeOH = 20: 1, silica gel) to give **ACK902** (12.4 mg, 0.028 mmol, 58%) as a white solid.

### 1.4 ACK903

2,5-dioxopyrrolidin-1-yl [1,1'-biphenyl]-4-carboxylate (44 mg, 0.15 mmol) was added to a solution of **9b** (10 mg, 0.05 mmol) and Et₃N (14 µL, 0.1 mmol) in DMSO (0.5 mL) and stirred for 8 h. After the reaction was complete, the reaction was extracted with EtOAc and H₂O. The combined organic layers were dried (MgSO₄), filtered and concentrated. The residue was purified by column chromatography (CH₂Cl₂ / MeOH = 20: 1, silica gel) to give **ACK903** (13.3 mg, 0.035 mmol, 69%) as a white solid.

### 1.5 ACK904

1-isothiocyanato-4-methoxybenzene (3.6 mg, 0.055 mmol) was added to a mixture of 9b (10 mg, 0.05 mmol) and Et₃N (14 µL, 0.1 mmol) in DMSO (0.5 mL) and stirred for 1 h. After the reaction was complete, the reaction was extracted with EtOAc and H₂O. The combined organic layers were dried (MgSO₄), filtered and concentrated. The residue was purified by column chromatography (CH₂Cl₂ / MeOH = 20: 1, silica gel) to give **ACK904** (9.5 mg, 0.026 mmol, 52%) as a white solid.

### 1.6 ACK905

Isothiocyanatobenzene (7.4 mg, 0.055 mmol) was added to a mixture of **9b** (10 mg, 0.05 mmol) and Et₃N (14 µL, 0.1 mmol) in DMSO (0.5 mL) and stirred for 1 h. After the reaction was complete, the reaction was extracted with EtOAc and H₂O. The combined organic layers were dried (MgSO₄), filtered and concentrated. The residue was purified by column chromatography (CH₂Cl₂ / MeOH = 20:1, silica gel) to give **ACK905** (8.4 mg, 0.025 mmol, 50%) as a white solid.

### 1.7 ACK906

4-isothiocyanato-4'-propyl-1,1'-biphenyl (13.9 mg, 0.055 mmol) was added to a mixture of **9b** (10 mg, 0.05 mmol) and Et₃N (14 µL, 0.1 mmol) in DMSO (0.5 mL) and stirred for 1 h. After the reaction was complete, the reaction was extracted with EtOAc and H₂O. The combined organic layers were dried (MgSO₄), filtered and concentrated. The residue was purified by column chromatography (CH₂Cl₂ / MeOH = 20: 1, silica gel) to give **ACK906** (14.6 mg, 0.032 mmol, 64%) as a white solid.

### 1.8 ACK907

4-hexyl-4'-isothiocyanato-1,1'-biphenyl (16.2 mg, 0.055 mmol) was added to a mixture of **9b** (10 mg, 0.05 mmol) and Et₃N (14 µL, 0.1 mmol) in DMSO (0.5 mL) and stirred for 1 h. After the reaction was complete, the reaction was extracted with EtOAc and H₂O. The combined organic layers were dried (MgSO₄), filtered and concentrated. The residue was purified by column chromatography (CH₂Cl₂ / MeOH = 20: 1, silica gel) to give **ACK907** (17.4 mg, 0.035 mmol, 70%) as a white solid.

### Example 2 In vitro study of the compound of Example 1

### 2.1 Inhibitory and selectivity on α-hGMII or α-hLM

The inhibitory activity (IC₅₀) of the compounds of example 1 against α-hGMII or α-hLM activity was performed in accordance with procedures as described in the "Material and Method" section. Results are summarized in Table 1.

**Table 1 Inhibitory potency and selectivity of the compound of Example 1 on α-hGMII or α-hLM**

| Compound | IC₅₀ (µM) | | Selectivity Index (SI) |
|---|---|---|---|
| | α-hLM | α-hGMII | |
| Swainsonine | 0.08 | 0.04 | 2 |
| ACK900 | 14 | 0.1 | 140 |
| ACK901 | 21.5 | 1 | 21.5 |
| ACK902 | 6.8 | 1.6 | 4.3 |
| ACK903 | 0.65 | 0.19 | 3.4 |
| ACK904 | 0.95 | 0.8 | 1.2 |
| ACK905 | 1.5 | 1.3 | 1.1 |

It was found that, ACK900, ACK901, ACK902, ACK903, and ACK905 were all capable of suppressing the activity of α-hGMII or α-hLM, with respective IC₅₀ being in the range between 0.1 to 21.5 µM. However, except for compounds ACK904 and ACK905, the rest of the compounds, i.e., ACK900, ACK901, ACK902 and ACK903, were found to exhibit selectivity toward α-hGMII than α-hLM, with a selective index (SI) between 3.4 to 140 (i.e., the compound is at least 3.4 to 140 times more selective to α-hGMII than to α-hLM).

### 2.2 N-glycosylation pattern in cellular level

Theoretically, an α-hGMII inhibitor would result in decreased formation of complex glycan and increased biosynthesis of hybrid glycan **(****FIG 1A****).** Accordingly, the effect of the compound of Example 1 (e.g., **ACK900)** on N-glycosylation in HepG2 or Huh7 cells was investigated in this example.

To this purpose, HepG2 and Huh7 cells were treated with **ACK900** in dose-dependent manner (0.1 to 10 µM) for 72h and N-glycan profiling was determined using mass spectrometric analysis. **(****FIGs 1B** and **1C****)).** This method was based on matching the experimental masses with the predicted masses of the glycans from the CFG carbohydrate database. The individual glycoforms were quantified and calculated for their proportions in all the glycoforms. Most N-glycans of untreated HepG2 and Huh7 were dominated by complex-type glycans containing sialic acids and fucoses. High-mannose or hybrid-type glycans were found in a relatively low percentage. However, applying **ACK900** to both cells lead to significant shift of N-glycopatterns from complex chains to the hybrid-type glycan. These glycoform data from MS analysis were consistent with the surface binding of lectin using fluorescence microscopy. The surface binding of Concanavalin A (ConA), a mannose-binding lectin, was markedly increased on these two cells treated with **ACK900** (10 µM) for 48h indicating that mannose-containing glycans on the cell surface were upregulated by α-hGMII inhibition of **ACK900** **(FIGs 1D** and **1F****).** On the other hand, the surface binding of galectin-1 (Gal1), a β-galactose binding protein supporting tumor-cell survival and often overexpressed in liver cancer cell, substantially decreased suggesting that **ACK900** experiment resulted in significant reduction of surface complex N-glycans recognizable by Gal1 **(FIGs1E and 1G****).**

Taken together, these data demonstrate that **ACK900** may modulate and remodel aberrant glycosylation of HCC which correlates with cancer progression.

### 2.3 Anti-proliferative, anti-migration and anti-invasion activities

The inhibitory activity (IC₅₀) of the compounds of example 1 on the growth of various cancer cell lines, including liver cancer cells (HepG2 and Huh7), pancreatic cancer cells (PANC-1), lung cancer cells (H1975) and triple-negative breast cancer cells (MDA-MB-231), in MTT assays were performed in accordance with procedures as described in the "Material and Method" section. Results are summarized in Table 2.

**Table 2 Anti-proliferative activity**

| **Compound** | **HepG2 IC₅₀ (µM)** | **Huh7 IC₅₀ (µM)** | **PANC-1 IC₅₀ (µM)** | **H1975 IC₅₀ (µM)** | **MDA-MB-231 IC₅₀ (µM)** |
|---|---|---|---|---|---|
| **ACK900** | 5.9 | 7.8 | 14.5 | 13.9 | 4.6 |
| **ACK901** | 1.8 | 1.3 | 0.3 | 7.7 | 1 |
| **ACK902** | 14.5 | 3.7 | 1.6 | 11.5 | 9.9 |
| **ACK906** | -^{b} | -^{b} | -^{b} | 19.1 | 8.3 |
| **ACK907** | -^{b} | -^{b} | -^{b} | 8.9 | 3.7 |
| **Sorafenib** | 7.42^{a} | 5.97^{a} | -^{b} | -^{b} | -^{b} |
| **Doxorubicin** | -^{b} | -^{b} | -^{b} | -^{b} | 1.3~3.0 |

| | | | | | |
|---|---|---|---|---|---|
| [a] Data from Int. J. Med. Sci. 2017, 14, 523. [b] - " not determined". | | | | | |

It was evident from the data in Table 2 that the present compounds **ACK900, ACK901,** and **ACK902** significantly reduced cell viability in a dose-dependent manner compared with untreated cells in all cancer cell lines with the half maximum inhibitory concentration (IC₅₀) in the range of 0.3-19.1 µM. Comparing to current drug sorafenib, the anti-proliferation activity of **ACK900** was promising (IC₅₀ = 5.9 µM in HepG2 and 7.8 µM in Huh7). Surprisingly, both **ACK901** and **ACK902** exhibited even low IC₅₀ values against cancer cells which more significant than clinical drug sorafenib or doxorubicin.

In addition to MTT assay, transwell assays revealed that the migratory **(****FIGs 2A** to 2G) and invasive **(****FIGs 3A** to **3G****)** capabilities of liver and pancreatic cancer cells were remarkably decreased after treatment with **ACK900, ACK901** or **ACK902.**

### Example 3 In vivo study of the compound of Example 1

*In vivo* tumor growth inhibition studies in human Huh7 xenograft rodents were performed in accordance with procedures as described in the "Material and Method" section. Xenograft tumors were generated by implanting Huh7 cells (5x10⁶) subcutaneously into the left and right flanks of each NOD-SCID mouse. Starting on the 7th day after the inoculation of Huh7 cells, the mice were injected with **ACK900, ACK901, ACK902** or sorafenib intraperitoneally (i.p.) at a dose of 30 mg/kg and twice a week for 34 days.

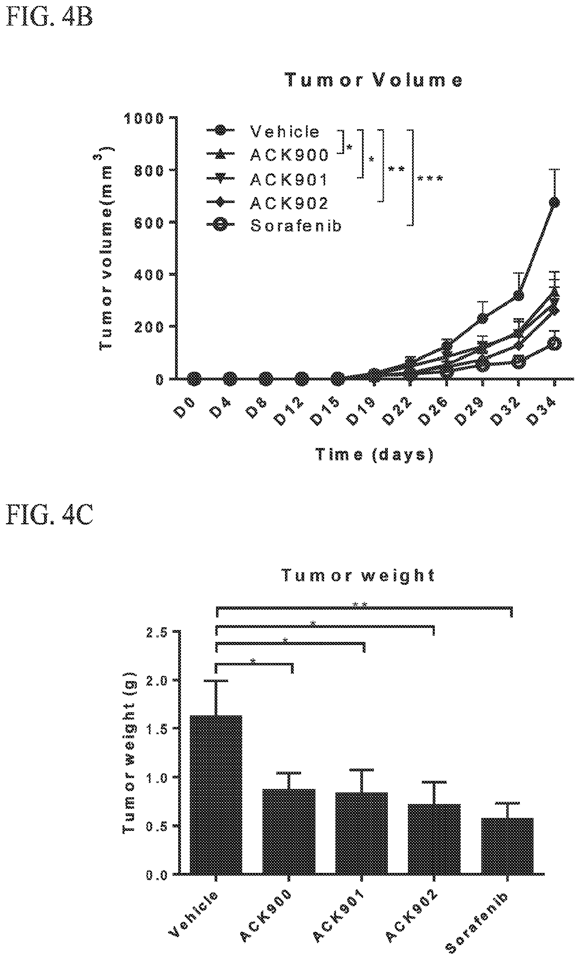

During treatment, none of the mice injected with **ACK900-ACK902** or sorafenib exhibited signs of harm or decreased body weight **(****FIG 4A****).** Significant reduction in tumor volume was found when the mice were administered with **ACK900-ACK902** or sorafenib (P<0.05) compared to that of the control **(****FIGs 4B and 4C****).** In addition, no serious side effects including hepatoxicity and nephrotoxicity were observed **(Table 3).**

**Table 3 Serum biochemistry test results**

| | **ALT (U/L)** | **AST (U/L)** | **BUN (mg/dl)** | **CRE (mg/dl)** | **TBIL (mg/dl)** |
|---|---|---|---|---|---|
| **Control (n=6)** | 117.60±95.17 | 200.16±114.04 | 20.06±6.01 | 0.4±0.07 | 0.98±0.98 |
| **ACK900 (n=7)** | 101.57±70.63 | 223.42±157.00 | 22.37±4.41 | 0.33±0.05 | 0.55±0.18 |
| **ACK901 (n=7)** | 74.85±82.13 | 173.85±95.67 | 21.87±3.77 | 0.47±0.18 | 0.64±0.37 |
| **ACK902 (n=7)** | 64.42±57.49 | 250±182.44 | 23.87±4.30 | 0.43±0.11 | 0.9±0.56 |
| **Sorafenib (n=6)** | 61.33±54.45 | 135.33±90.76 | 23±2.53 | 0.35±0.19 | 0.73±0.32 |

| | | | | | |
|---|---|---|---|---|---|
| Note: ALT, Alanine Aminotransferase; AST, Aspartate Aminotransferase; BUN, Blood Urea Nitrogen; CRE, Creatinine; TBIL, Total Bilirubin. | | | | | |

Taken together, the results in this example support the hypothesis that **ACK** series molecules may serve as candidate compounds for the development of novel anti-tumor agents.

## Claims

1. A compound, which is selected from the group consisting of:

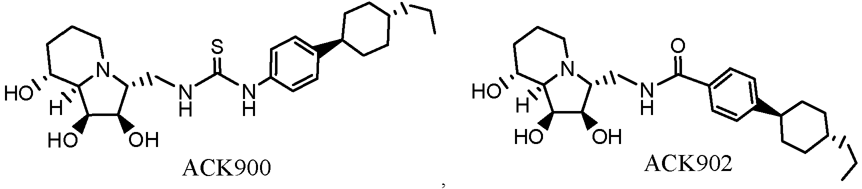

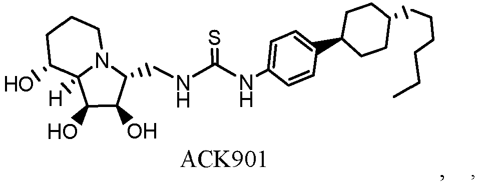

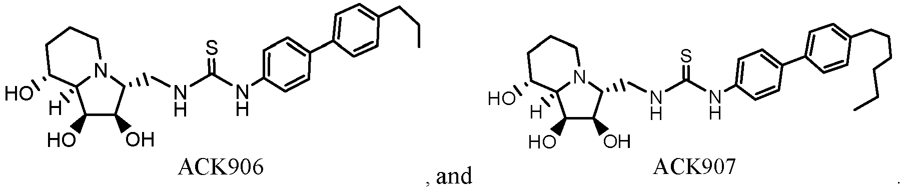

2. A compound having the structure of,

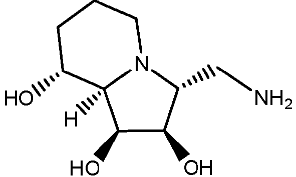

3. A pharmaceutical composition comprising a compound of claim 1, or a pharmaceutically acceptable salt thereof, and a pharmaceutically acceptable excipient.

4. The pharmaceutical composition of claim 3 for use in the treatment of a subject having a cancer.

5. The pharmaceutical composition of claim 3 for use of claim 4, wherein the compound is selected from the group consisting of 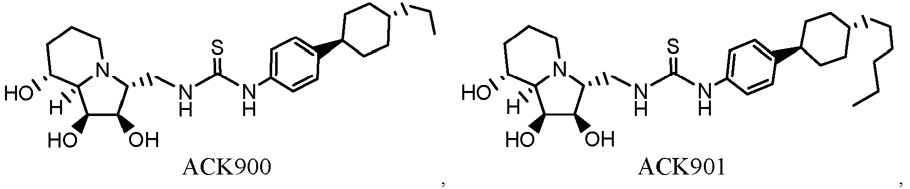 and

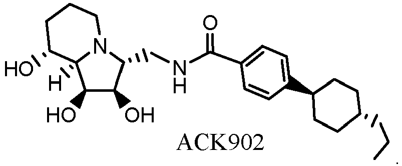

6. The pharmaceutical composition of claim 3 for use of claim 4, wherein the cancer is selected from the group consisting of bone tumor, brain cancer, breast cancer, cervical cancer, CNS neoplasm, colon cancer, esophageal cancer, Ewing's sarcoma, head and neck cancer, Hodgkin's disease, larynx cancer, leukemia, liver cancer, lymphoma, melanoma, multiple myeloma, nasopharynx cancer, neuroblastoma, non-small-cell lung (NSCL) cancer, pancreatic cancer, prostate cancer, rectal cancer, retinoblastoma, small-cell lung (SCL) cancer, testicular cancer, thyroid cancer, skin cancer other than melanoma, and Wilms' tumor.

7. The pharmaceutical composition of claim 3 for use of claim 6, wherein the cancer is a metastatic cancer.

8. The pharmaceutical composition of claim 3 for use of claim 4, wherein the subject is a mammal.

## Patentansprüche

1. Verbindung, die aus der Gruppe ausgewählt ist, bestehend aus:

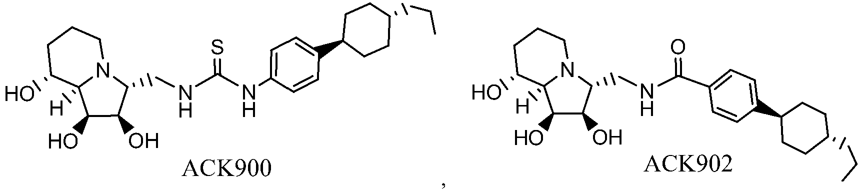

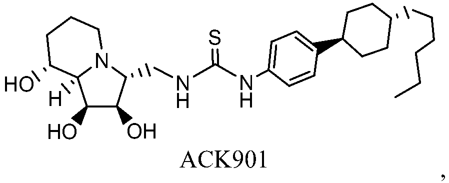

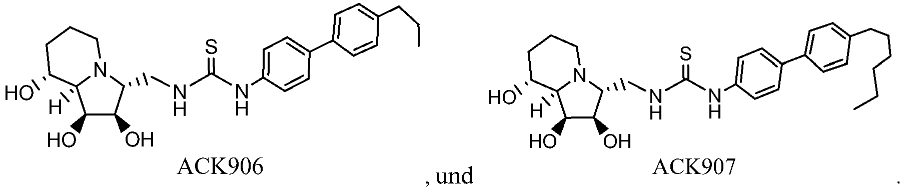

2. Verbindung mit der Struktur gemäß

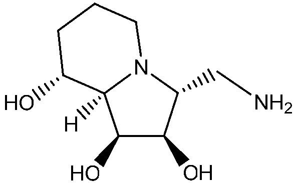

3. Pharmazeutische Zusammensetzung, welche eine Verbindung nach Anspruch 1 oder ein pharmazeutisch verträgliches Salz davon, sowie einen pharmazeutisch verträglichen Hilfsstoff umfasst.

4. Pharmazeutische Zusammensetzung nach Anspruch 3 zur Verwendung bei der Behandlung eines Subjekts, welches Krebs hat.

5. Pharmazeutische Zusammensetzung nach Anspruch 3 zur Verwendung nach Anspruch 4, wobei die Verbindung aus der Gruppe ausgewählt ist, bestehend aus 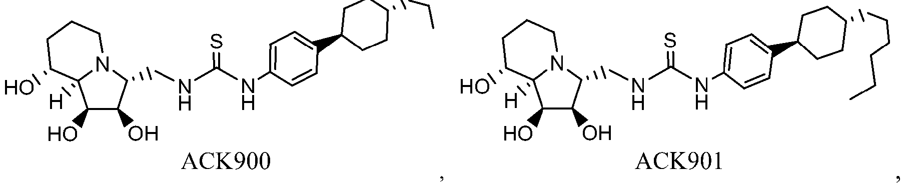 und

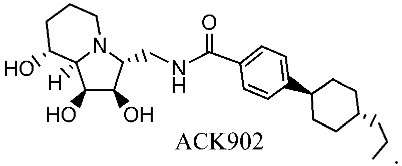

6. Pharmazeutische Zusammensetzung nach Anspruch 3 zur Verwendung nach Anspruch 4, wobei der Krebs aus der Gruppe ausgewählt ist, die aus Knochentumor, Hirntumor, Brustkrebs, Gebärmutterhalskrebs, ZNS-Neoplasma, Darmkrebs, Speiseröhrenkrebs, Ewing-Sarkom, Kopf- und Hals-Krebs, Hodgkin-Krankheit, Kehlkopfkrebs, Leukämie, Leberkrebs, Lymphom, Melanom, Multiples Myelom, Nasopharynxkarzinom, Neuroblastom, nicht-kleinzelliger Lungenkrebs (NSCL), Bauchspeicheldrüsenkrebs, Prostatakrebs, Rektumkrebs, Retinoblastom, kleinzelliger Lungenkrebs (SCL), Hodenkrebs, Schilddrüsenkrebs, Hautkrebs, der von Melanomen verschieden ist, und Wilms-Tumor besteht.

7. Pharmazeutische Zusammensetzung nach Anspruch 3 zur Verwendung nach Anspruch 6, wobei der Krebs ein metastasierender Krebs ist.

8. Pharmazeutische Zusammensetzung nach Anspruch 3 zur Verwendung nach Anspruch 4, wobei das Subjekt ein Säugetier ist.

## Revendications

1. Composé qui est sélectionné à partir du groupe consistant en :

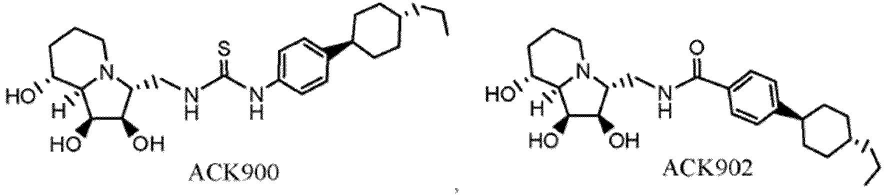

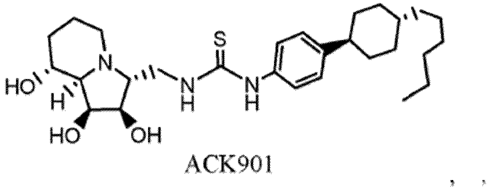

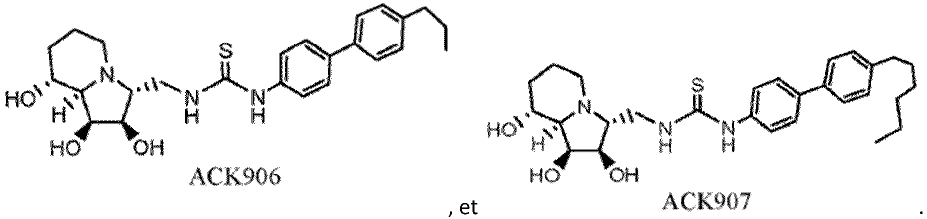

2. Composé présentant la structure de

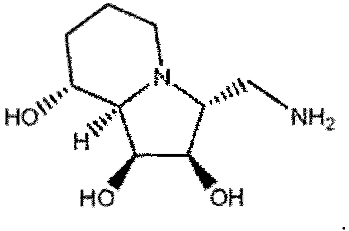

3. Composition pharmaceutique comprenant un composé selon la revendication 1, ou un sel pharmaceutiquement acceptable de celui-ci, et un excipient pharmaceutiquement acceptable.

4. Composition pharmaceutique selon la revendication 3 pour une utilisation dans le traitement d'un sujet atteint d'un cancer.

5. Composition pharmaceutique selon la revendication 3 pour une utilisation selon la revendication 4, dans laquelle le composé est sélectionné à partir du groupe consistant en 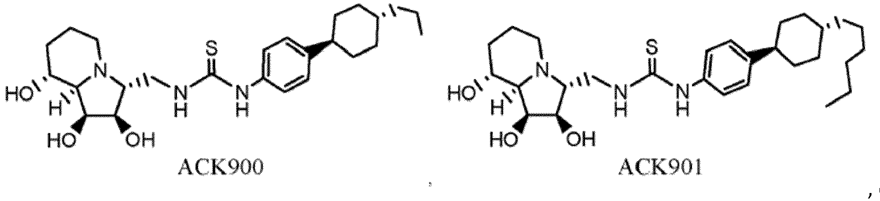 et

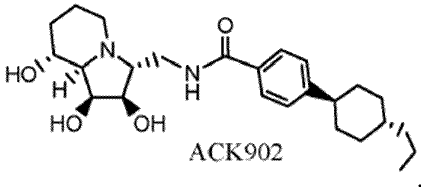

6. Composition pharmaceutique selon la revendication 3 pour une utilisation selon la revendication 4, dans laquelle le cancer est sélectionné à partir du groupe consistant en la tumeur osseuse, le cancer du cerveau, le cancer du sein, le cancer du col de l'utérus, le néoplasme du SNC, le cancer du côlon, le cancer de l'œsophage, le sarcome d'Ewing, le cancer de la tête et du cou, la maladie de Hodgkin, le cancer du larynx, la leucémie, le cancer du foie, le lymphome, le mélanome, le myélome multiple, le cancer du nasopharynx, le neuroblastome, le cancer du poumon non à petites cellules (NSCL), le cancer du pancréas, le cancer de la prostate, le cancer du rectum, le rétinoblastome, le cancer du poumon à petites cellules (SCL), le cancer des testicules, le cancer de la thyroïde, le cancer de la peau autre que le mélanome et la tumeur de Wilms.

7. Composition pharmaceutique selon la revendication 3 pour une utilisation selon la revendication 6, dans laquelle le cancer est un cancer métastatique.

8. Composition pharmaceutique selon la revendication 3 pour une utilisation selon la revendication 4, dans laquelle le sujet est un être mammifère.
